# EUROPEAN PATENT APPLICATION

(11) **EP 1 520 856 A1**
(43) Date of publication of application: **06.04.2005**
(21) Application number: 03760911.2
(22) Date of filing: 19.06.2003
(51) Int. Cl.: C07D 221/16, C07D 401/12, C07D 405/12, C07D 409/12, C07D 491/048, A61K 31/473, A61K 31/4741, A61K 31/497, A61P 7/06, A61P 15/00, A61P 15/08, A61P 15/10, A61P 15/14, A61P 19/10, A61P 35/00, A61P 43/00

(54) **ANDROGEN RECEPTOR AGONIST**

(30) Priority: 19.06.2002 JP 2002179088
(71) Applicant: Kaken Pharmaceutical Co., Ltd., Tokyo 113-8650 (JP)
(72) Inventor: MIYAKAWA, Motonori, c/o Kaken Pharma. Co. Ltd, Kyoto-shi, Kyoto 607-8042 (JP); SUMITA, Yuji, c/o Kaken Pharma. Co. Ltd, Kyoto-shi, Kyoto 607-8042 (JP); FURUYA, Kazuyuki, c/o Kaken Pharma. Co. Ltd, Kyoto-shi, Kyoto 607-8042 (JP); ICHIKAWA, Kiyonoshin, c/o Kaken Pharma. Co. Ltd, Kyoto-shi, Kyoto 607-8042 (JP); YAMAMOTO, Noriko, c/o Kaken Pharma. Co. Ltd, Kyoto-shi, Kyoto 607-8042 (JP); HANADA, Keigo, Shiga 525-0023 (JP); AMANO, Seiji, c/o Kaken Pharma. Co. Ltd, Kyoto-shi, Kyoto 607-8042 (JP); NEJISHIMA, Hiroaki, c/o Kaken Pharma. Co. Ltd, Kyoto-shi, Kyoto 607-8042 (JP)
(74) Representative: HOFFMANN EITLE
(86) International application number: PCT/JP2003/007799
(87) International publication number: WO 2004/000816

(57) **Abstract**

The present invention provides nonsteroidal tetrahydroquinoline derivatives of general formula (I) or salts thereof: 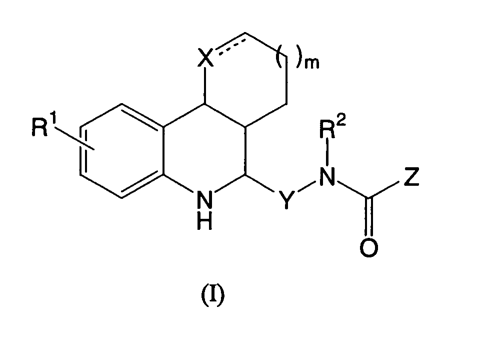 (R¹, R², X, Y, Z and m are as defined in claim 1) which show no excessive action on the prostate but show a particularly potent androgen receptor agonistic action on skeletal muscle tissue and bone tissue, as well as pharmaceuticals comprising such derivatives or their salts as active ingredients.

## Description

### TECHNICAL FIELD

This invention relates to tetrahydroquinoline derivatives or salts thereof which do not exhibit excessive action on the prostate but which exhibit particularly strong androgen receptor agonism on skeletal muscle tissue and bone tissue, and pharmaceuticals containing such derivatives or their salts.

### BACKGROUND ART

Androgens are a generic name for C19 steroids. They are sex hormones important for the normal sexual differentiation and growth of males, masculinization at puberty, activation of initial spermatogenesis in the testes, and maintenance of male function. About 90% of androgens are produced by Leydig cells of the testes, the remaining 10% by the adrenal gland, mainly as testosterone, and secreted into the blood. Testosterone is taken up into target cells, and converted by 5α-reductase into dihydrotestosterone (DHT) with potent biological activity. DHT, as well as testosterone, plays an important role in the development of male secondary sex characteristics (growth of sebaceous glands, acne, development of body hair, voice deepening, development of beards), growth of external genitalia (penis, testis), growth of sex accessory organs (prostate, seminal vesicles), sexual impulse, and occurrence of erection.

Aside from these major actions, androgens have actions other than those on the reproductive system, such as protein anabolic action (e.g., increases in skeletal muscles and bone mass, and acceleration of erythropoiesis promoting action), and suppression of gonadotropin secretion. Target cells for androgens are not only localized in the tissues of external genitalia and sex accessory organs but are also widely distributed in the brain, pituitary gland, muscular tissues, bones, and kidneys (N. Engl. J. Med. 334, 707-714, 1996).

In addition to these roles, androgens are reported to show an anti-inflammatory action. Nowadays, it is becoming clear that androgens attenuate arthritis and autoimmune disease by inhibiting the proliferation of inflammatory cells or suppressing the production of cytokines such as IL-6 (Ann. Rheum. Dis. 55, 811-815, 1996).

All androgenic actions are mediated through androgen receptor (hereinafter referred to as AR) having a molecular weight of about 100,000 which is present in the nuclei of target cells. The gene of AR was cloned by Chang et al. and Lubahn et al. in 1988. Their studies demonstrated that AR has a similar structure to estrogen, progesterone, mineral corticoid and glucocorticoid receptors and they build a family of nuclear steroid receptors (Science 240, 324-326, 327-330, 1988). Androgens with high liposolubility penetrate the target cell membrane by passive diffusion, and bind to the hormone-binding region of AR specifically and with high affinity to form dimers, which bind to an androgen responsive DNA region (androgen response element: ARE) localized upstream from a particular gene. As a result, transcription of the target gene is initiated to induce the expression of mRNA, thereby producing a functional protein responsible for an androgenic action, thus exhibiting this action (Trend in Endocrinology and Metabolism 9, 317-324, 1998). In connection with this mechanism, compounds which bind to AR and show similar actions to those of natural ligands such as testosterone are defined as agonists.

As the AR agonists, androgen steroid preparations such as testosterone esters and other derivatives are currently used in the treatment of male hypogonadism, wasting diseases (derived from malignant tumor, trauma, chronic renal disease, or burns), and osteoporosis.

However, these androgen steroid preparations can cause side effects inherent in steroid preparations, such as hepatic dysfunction and gastrointestinal disorder, and may develop androgen-dependent tumor (e.g. prostatic cancer) or prostatic hypertrophy, or aggravate symptoms of these diseases, because they act excessively on the prostate if administered to male patients, especially to elderly patients. If these preparations are administered to female patients, they pose major problems of virilizing actions, such as changes in the vocal cord (male-like hoarseness), hypertrichosis of the body trunk, alopecia and acne.

Hence, nonsteroidal AR agonists which do not show excessive action on the prostate and are minimal in side effects are desired for the treatment of hypogonadism and have been the subject of ongoing R&D efforts. However, globally recognized compounds are yet to be created.

In the case of drugs indicated for wasting disease and osteoporosis, compounds which exhibit particularly potent AR agonism on skeletal muscle tissue and bone tissue are desired but no such compounds are yet to be created.

### DISCLOSURE OF THE INVENTION

The present invention has been accomplished in view of the research on the prevention and treatment of such AR mediated diseases. An object of the present invention is to provide novel nonsteroidal compounds or salts thereof that do not exhibit excessive action on the prostate as is often observed with androgen steroid preparations but which exhibit particularly strong AR agonism on skeletal tissue and bone tissue. Another object of the present invention is to provide pharmaceuticals comprising such compounds or salts thereof as an active ingredient.

The inventors of the present invention conducted in-depth studies in an attempt to attain the above objects. As a result, they have found that among various tetrahydroquinoline derivatives, specified compounds of the following formula (I) (hereinafter referred to as "compounds of the present invention") do not act excessively on the prostate but exhibit particularly strong AR agonism on skeletal muscle tissue and bone tissue. Based on these findings, they have accomplished this invention.

That is, according to one embodiment, the present invention relates to a tetrahydroquinoline derivative represented by the following formula (I) or pharmacologically acceptable salts thereof: 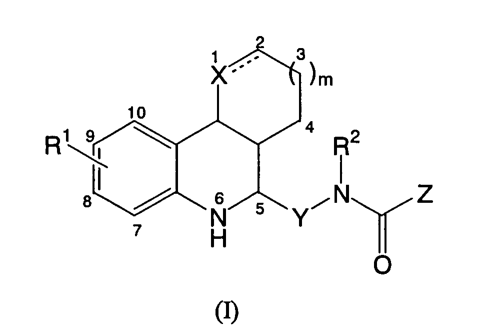 (numbers 1 - 10 used in the above formula (I) assume the case where m is 1 and if m is 0, the position of number 3 is absent, so numbers 4 - 10 are replaced by numbers 3 - 9 to indicate the respective positions for the purpose of the following explanation)
wherein R¹ represents a nitro group or a cyano group;
X represents CH or O, provided that when X is CH, the dashed line represents a double bond;
m represents 0 or 1;
Y represents an alkylene group having 1 - 5 carbon atoms which may be substituted by a substituent selected from the group consisting of an alkyl group having 1 - 5 carbon atoms and a cycloalkyl group having 3 - 7 carbon atoms;
R² represents a hydrogen atom, an alkyl group having 1 - 5 carbon atoms, a cycloalkyl group having 3 - 7 carbon atoms or an aralkyl group having 7 - 9 carbon atoms;
Z represents -B-O-Q
[wherein B represents an alkylene group having 1 - 5 carbon atoms which may be substituted by a substituent selected from the group consisting of an alkyl group having 1 - 5 carbon atoms and a cycloalkyl group having 3 - 7 carbon atoms; Q is a hydrogen atom, an alkyl group having 1 - 5 carbon atoms or a cycloalkyl group having 3 - 7 carbon atoms which may be substituted by a substituent selected from the group consisting of a halogen atom, a hydroxyl group, a cyano group and an alkoxy group having 1 - 5 carbon atoms, or an aryl group, a heteroaryl group or an aralkyl group having 7 - 9 carbon atoms which may have a substituent R³,
R³ represents an alkyl group having 1 - 5 carbon atoms which may be substituted by a fluorine atom, a halogen atom, an aryl group, a heteroaryl group, a nitro group, a cyano group, -A-R⁴ {wherein A represents -CO-, -CO₂-, -COS-, -CONR⁵-, -O-, -OCO-, -OSO₂-, -S-, -SCO-, -SO-, -SO₂-, -NR⁵-, -NR⁵CO-, -NR⁵SO₂-, -NR⁵CONH-, -NR⁵CSNH- or -NR⁵COO- (wherein R⁵ represents a hydrogen atom, an alkyl group having 1 - 5 carbon atoms, a cycloalkyl group having 3 - 7 carbon atoms or an aralkyl group having 7 - 9 carbon atoms), R⁴ is a hydrogen atom, an alkyl group having 1 - 5 carbon atoms which may be substituted by a fluorine atom, a cycloalkyl group having 3 - 7 carbon atoms, a halogen atom, or an aryl group or a heteroaryl group which may be substituted by R⁶ (wherein R⁶ represents an alkyl group having 1 - 5 carbon atoms, an alkoxy group having 1 - 5 carbon atoms or a halogen atom), provided that when A is -NR⁵- or -CONR⁵-, R⁴ and R⁵ may, together with the nitrogen atom to which they are bonded, form pyrrolidine or piperidine)}, or -A'-(CH₂)ₙ-R^{4'} {wherein A' represents a single bond, -CO-, -CO₂-, -COS-, -CONR^{5'}-, -O-, -OCO-, -OSO₂-, -S-, -SCO-, -SO-, -SO₂-, -NR^{5'}-, -NR^{5'}CO-, -NR^{5'}SO₂-, -NR^{5'}CONH-, -NR^{5'}CSNH- or -NR^{5'}COO- (wherein R^{5'} represents a hydrogen atom, an alkyl group having 1 - 5 carbon atoms, a cycloalkyl group having 3 - 7 carbon atoms or an aralkyl group having 7 - 9 carbon atoms), n represents an integer of 1 or 2, R^{4'} represents a hydrogen atom, an alkyl group having 1 - 5 carbon atoms which may be substituted by a fluorine atom, a cycloalkyl group having 3 - 7 carbon atoms, a halogen atom, a hydroxyl group, a cyano group, an alkoxy group having 1 - 5 carbon atoms, an alkylacyloxy group having 2 - 5 carbon atoms, an alkoxycarbonyl group having 2 - 5 carbon atoms, an aryl group or a heteroaryl group which may be substituted by R^{6'} (wherein R^{6'} represents an alkyl group having 1 - 5 carbon atoms, an alkoxy group having 1 - 5 carbon atoms or a halogen atom), or -NR^{7'}R^{8'} (wherein R^{7'} and R^{8'} each independently have the same meaning as the aforementioned R^{5'}, provided that R^{7'} and R^{8'} may, together with the nitrogen atom to which they are bonded, form pyrrolidine or piperidine), provided that when A' is -NR^{5'}- or -CONR^{5'}-, R^{4'} and R^{5'} may, together with the -N-(CH₂)ₙ- to which they are bonded, form pyrrolidine or piperidine}], or alternatively Z represents -(CH₂)ᵣ-W
[wherein r represents an integer of 0 - 2, W represents a phenyl group having substituent R⁹ at p-position, a naphthyl group which may have substituent R¹⁰ or a heteroaryl group which may be substituted by 1 - 3 independent R¹¹'s (wherein R⁹, R¹⁰ and R¹¹ independently have the same meaning as the aforementioned R³)]. The present invention also relates to a pharmaceutical and an androgen receptor agonist, each comprising the tetrahydroquinoline derivative of the formula (I) or pharmacologically acceptable salts thereof as the active ingredient.

### BRIEF DESCRIPTION OF THE DRAWINGS

In the drawings, white bars represent data for the case where no test substance was included; the single asterisk (*) shows a significant increase at p < 5% as compared with ORX control; the double asterisk (**) shows a significant increase at p < 1% as compared with ORX control.
Fig. 1 is a graph showing the effect of a compound of the present invention (the compound of Example 1) on the weight of the prostate as compared with dihydrotestosterone (DHT);
Fig. 2 is a graph showing the effect of a compound of the present invention (the compound of Example 1) on the femoral bone mineral density as compared with DHT;
Fig. 3 is a graph showing the effect of a compound of the present invention (the compound of Example 1) on the weight of the levator ani muscle as compared with DHT;
Fig. 4 is a graph showing the effect of compounds of the present invention (the compounds of Examples 12 and 65) on the weight of the prostate as compared with DHT;
Fig. 5 is a graph showing the effect of compounds of the present invention (the compounds of Examples 12 and 65) on the femoral bone mineral density as compared with DHT;
Fig. 6 is a graph showing the effect of compounds of the present invention (the compounds of Examples 12 and 65) on the weight of the levator ani muscle as compared with DHT;
Fig. 7 is a graph showing the effect of compounds of the present invention (the compounds of Examples 113 and 114) on the weight of the prostate as compared with DHT;
Fig. 8 is a graph showing the effect of compounds of the present invention (the compounds of Examples 113 and 114) on the femoral bone mineral density as compared with DHT;
Fig. 9 is a graph showing the effect of compounds of the present invention (the compounds of Examples 113 and 114) on the weight of the levator ani muscle as compared with DHT;
Fig. 10 is a graph showing the effect of an orally administered compound of the present invention (the compound of Example 1) on the weight of the prostate as compared with subcutaneously administered testosterone propionate (TP);
Fig. 11 is a graph showing the effect of an orally administered compound of the present invention (the compound of Example 1) on the femoral bone mineral density as compared with subcutaneously administered TP;
Fig. 12 is a graph showing the effect of an orally administered compound of the present invention (the compound of Example 1) on the weight of the levator ani muscle as compared with subcutaneously administered TP;
Fig. 13 is a graph showing the effect of a compound of the present invention (the compound of Example 108) and methyl testosterone (MT), both administered orally, on the weight of the prostate;
Fig. 14 is a graph showing the effect of a compound of the present invention (the compound of Example 108) and MT, both administered orally, on the femoral bone mineral density;
Fig. 15 is a graph showing the effect of a compound of the present invention (the compound of Example 108) and MT, both administered orally, on the weight of the levator ani muscle;
Fig. 16 is a graph showing the effect of a compound of the present invention (the compound of Example 129) and MT, both administered orally, on the weight of the prostate;
Fig. 17 is a graph showing the effect of a compound of the present invention (the compound of Example 129) and MT, both administered orally, on the femoral bone mineral density; and
Fig. 18 is a graph showing the effect of a compound of the present invention (the compound of Example 129) and MT, both administered orally, on the weight of the levator ani muscle.

### BEST MODE FOR CARRYING OUT THE INVENTION

According to another embodiment of the present invention, there are provided tetrahydroquinoline derivatives of the formula (I) where Y is -CH(CH₃)-CH₂- or -C(CH₃)₂-CH₂-, X is CH, m is 0, R² is a hydrogen atom and Z is -CH₂-O-Q (wherein Q represents an alkyl group having 1 - 5 carbon atoms) or pharmacologically acceptable salts thereof.

According to yet another embodiment of the present invention, there are provided tetrahydroquinoline derivatives of the formula (I) where Y is -CH(CH₃)-CH₂- or -C(CH₃)₂-CH₂-, m is 0, R² is a hydrogen atom and Z is -W [wherein W is a heteroaryl group which may be substituted by 1 - 3 independent R¹¹'s or a phenyl group having substituent R⁹ at p-position {wherein R¹¹ and R⁹ independently represent a halogen atom, an alkyl group having 1 - 5 carbon atoms which may be substituted by a fluorine atom, a nitro group, a cyano group, -A-R⁴ (wherein A is -CO-, -CO₂-, -O-, -NHCO- or -NHCONH-, and R⁴ is a hydrogen atom or an alkyl group having 1 - 5 carbon atoms which may be substituted by a fluorine atom) or -A'-(CH₂)ₙ-R^{4'} (wherein A' is -CO-, -CO₂-, -O-, -NHCO- or -NHCONH-, R^{4'} is a hydrogen atom, an alkyl group having 1 - 5 carbon atoms which may be substituted by a fluorine atom, a hydroxyl group, a halogen atom or an alkoxy group having 1 - 5 carbon atoms, and n is an integer of 1 or 2)}] or pharmacologically acceptable salts thereof.

According to a further embodiment of the present invention, there are provided tetrahydroquinoline derivatives of the formula (I) where Y is -CH(CH₃)-CH₂- or -C(CH₃)₂-CH₂-, m is 0, R² is a hydrogen atom and Z is a phenyl group having substituent R⁹ at p-position or a heteroaryl group having substituent R¹¹ {wherein R⁹ and R¹¹ independently represent a halogen atom, -O-R⁴ or -NHCO-R⁴ (wherein R⁴ represents a hydrogen atom or an alkyl group having 1 - 5 carbon atoms which may be substituted by a fluorine atom)} or pharmacologically acceptable salts thereof.

According to a still further embodiment of the present invention, there are provided tetrahydroquinoline derivatives of the formula (I) where Y is -CH(CH₃)-CH₂- or -C(CH₃)₂-CH₂-, m is 0, R² is a hydrogen atom and Z is a phenyl group having substituent R⁹ at p-position or a heteroaryl group having substituent R¹¹ {wherein R⁹ and R¹¹ represent -NHCO-R⁴ (wherein R⁴ represents a hydrogen atom or an alkyl group having 1 - 5 carbon atoms which may be substituted by a fluorine atom)} or pharmacologically acceptable salts thereof.

The substituents in the formula (I) representing the compounds of the present invention will be described below.

Specific examples of the "alkyl group having 1 - 5 carbon atoms" include straight chain or branched chain alkyl groups, such as a methyl group, an ethyl group, a *n*-propyl group, an isopropyl group, a *n*-butyl group, an isobutyl group, a *tert*-butyl group, a *sec*-butyl group, a *n*-pentyl group, a *tert*-amyl group, a 3-methylbutyl group, and a neopentyl group.

Specific examples of the "cycloalkyl group having 3 - 7 carbon atoms" include a cyclopropyl group, a cyclobutyl group, a cyclopentyl group, a cyclohexyl group, and a cycloheptyl group.

Specific examples of "alkylene group having 1 - 5 carbon atoms" include a methylene group, an ethylene group, a tetramethylene group, and a pentamethylene group.

Specific examples of the "aralkyl group having 7 - 9 carbon atoms" include a benzyl group, a phenethyl group, and a phenylpropyl group.

Specific examples of the "aryl group" include a phenyl group and naphthyl groups (a 1-naphthyl group and a 2-naphthyl group).

Specific examples of the "halogen atom" include a fluorine atom, a chlorine atom, a bromine atom, and an iodine atom.

Specific examples of the "heteroaryl group" include a 2-pyridyl group, a 3-pyridyl group, a 4-pyridyl group, a 2-pyrazinyl group, a 2-pyrrolyl group, a 2-indolyl group, a 2-furyl group, a 3-furyl group, a 2-thienyl group, a 3-thienyl group, a 2-pyrrole group, and a 3-pyrrole group.

Specific examples of the "alkoxy group having 1 - 5 carbon atoms" include straight chain or branched chain alkoxy groups, such as a methoxy group, an ethoxy group, a *n*-propoxy group, an isopropoxy group, a *n*-butoxy group, an isobutoxy group, a *tert*-butoxy group, a *sec*-butoxy group, a *n*-pentyloxy group, a *tert*-amyloxy group, a 3-methylbutoxy group, and a neopentyloxy group.

Specific examples of the "alkylacyloxy group having 2 - 5 carbon atoms" include straight chain or branched chain alkylacyloxy groups, such as an acetoxy group, a propionyloxy group, a butyryloxy group, an isobutyryloxy group, a valeryloxy group, an isovaleryloxy group, and a pivaloyloxy group.

Specific examples of the "alkoxycarbonyl group having 2 - 5 carbon atoms" include straight chain or branched chain alkoxycarbonyl groups, such as a methoxycarbonyl group, an ethoxycarbonyl group, a n-propoxycarbonyl group, an isopropoxycarbonyl group, a n-butoxycarbonyl group, an isobutoxycarbonyl group, a tert-butoxycarbonyl group, and a sec-butoxycarbonyl group.

Referring to the "alkyl group having 1 - 5 carbon atoms which may be substituted by a fluorine atom", specific examples of the fluorine atom substituted alkyl group include a trifluoromethyl group, a 2,2,2-trifluoroethyl group, and a tetrafluoroethyl group.

Referring to the "alkoxy group having 1 - 5 carbon atoms which may be substituted by a fluorine atom", specific examples of the fluorine atom substituted alkoxy group include a trifluoromethoxy group, a 2,2,2-trifluoroethoxy group, and a tetrafluoroethoxy group.

Preferred modes for the compounds of the formula (I) include, for example, the following:
m is preferably 0;
   In the definition of Y, the number of the substituents by which the alkylene group having 1 - 5 carbon atoms may be substituted is preferably 1 - 3 and the preferred examples of the substituent include a methyl group and an ethyl group; a preferred example of Y is such that Y is -CH(CH₃)-CH₂- or -C(CH₃)₂-CH₂-;
R² is preferably a hydrogen atom;
R¹ is preferably substituted at 9-position when m is 1 (at 8-position when m is 0); the preferred example of R¹ is a cyano group;

When Z is -B-O-Q, X is preferably CH and B is preferably an alkylene group having 1 - 5 carbon atoms or an alkylene group having 1 - 5 carbon atoms which is substituted by 1 - 3 substituents (methyl or ethyl groups), with -CH₂- being more preferred; Q is preferably an alkyl group having 1 - 5 carbon atoms; in this case, R¹ is preferably a nitro group;

When Z is -(CH₂)ᵣ-W, it is preferred that r is 0 and W is a heteroaryl group which may be substituted by 1 - 3 independent R¹¹'s or a phenyl group having substituent R⁹ at p-position {wherein R¹¹ and R⁹ independently represent a halogen atom, an alkyl group having 1 - 5 carbon atoms which may be substituted by a fluorine atom, a nitro group, a cyano group, -A-R⁴ (wherein A is -CO-, -CO₂-, -O-, -NHCO- or -NHCONH-, and R⁴ is a hydrogen atom or an alkyl group having 1 - 5 carbon atoms which may be substituted by a fluorine atom) or -A'-(CH₂)ₙ-R^{4'} (wherein A' is -CO-, -CO₂-, -O-, -NHCO- or -NHCONH-, R^{4'} is a hydrogen atom, an alkyl group having 1 - 5 carbon atoms which may be substituted by a fluorine atom, a hydroxyl group, a halogen atom or an alkoxy group having 1 - 5 carbon atoms, and n is an integer of 1 or 2)}.

Preferred examples of the above heteroaryl group include a pyridyl group.

Specific examples of the above R⁹ and R¹¹ include a halogen atom, a methyl group, a trifluoromethyl group, a nitro group, a cyano group, -OCH₂CH₂Cl, -CO₂Me, -OMe, -OEt, -OCF₃, -OCH₂OMe, -OCH₂CH₂OH, -OCH₂CH₂OCOMe, -NHCOMe, -NHCOCF₃, -NHCOCH₂OH, -NHCOCH₂OCOMe, -NHCONHEt, and -NHCOH.

Preferably, R⁹ and R¹¹ are a halogen atom, -O-R⁴ or -NHCO-R⁴ (wherein R⁴ represents a hydrogen atom or an alkyl group having 1 - 5 carbon atoms which may be substituted by a fluorine atom), with -NHCO-R⁴ (wherein R⁴ represents a hydrogen atom or an alkyl group having 1 - 5 carbon atoms which may be substituted by a fluorine atom) being more preferred. Here, -OMe, -OEt and -OCF₃ may be given as examples of -O-R⁴. In addition, -NHCOMe, -NHCOCF₃ and -NHCOH may be given as examples of -NHCO-R⁴.

Preferred compounds of the present invention are as follows:
2-methoxy-*N*-[2-methyl-2-(8-nitro-3a,4,5,9b-tetrahydro-3*H*-cyclopenta[*c*]quinolin-4-yl)propyl]-1-acetamide;
4-bromo-*N*-[2-methyl-2-(8-nitro-2,3,3a,4,5,9b-hexahydro-furo[3,2-*c*]quinolin-4-yl)propyl]benzamide;
*N*-[2-methyl-2-(8-nitro-2,3,3a,4,5,9b-hexahydrofuro[3,2-*c*]quinolin-4-yl)propyl]-4-trifluoromethoxybenzamide;
*N*-[2-(8-cyano-2,3,3a,4,5,9b-hexahydro-furo[3,2-*c*]quinolin-4-yl)-2-methyl-propyl]-4-trifluoromethoxybenzamide;
*N*-[2-(8-cyano-2,3,3a,4,5,9b-hexahydro-furo[3,2-*c*]quinolin-4-yl)-2-methyl-propyl]-4-ethoxybenzamide;
6-acetylamino-*N*-[2-(8-cyano-3a,4,5,9b-tetrahydro-3H-cyclopenta[c]quinolin-4-yl)-2-methyl-propyl]nicotinamide;
4-acetylamino-*N*-[2-(8-cyano-2,3,3a,4,5,9b-hexahydrofuro[3,2-c]quinolin-4-yl)propyl]benzamide.

If asymmetric carbon is present in the compound of the present invention represented by the formula (I), its racemic forms, diastereomers, and individual optical isomers are all included in the present invention. If its geometrical isomers are present, (E) forms, (Z) forms, and mixtures of them are all included in the present invention.

The tetrahydroquinoline ring in the compound of the present invention represented by the formula (I) has three asymmetric carbons, giving rise to diastereomers.

The compound of the present invention has the following preferred isomers:
(3a*R**,4*S**,9b*S**)-2-methoxy-*N*-[2-methyl-2-(8-nitro-3a,4,5,9b-tetrahydro-3*H*-cyclopenta[*c*]quinolin-4-yl)propyl]-1-acetamide (the compound of Example 1);
(3a*S**,4*S**,9b*S**)-4-bromo-*N*-[2-methyl-2-(8-nitro-2,3,3a,4,5,9b-hexahydro-furo[3,2-*c*]quinolin-4-yl)propyl]benzamide (the compound of Example 12);
(3a*S**,4*S**,9b*S**)-*N*-[2-methyl-2-(8-nitro-2,3,3a,4,5,9b-hexahydro-furo[3,2-*c*]quinolin-4-yl)propyl]-4-trifluoromethoxybenzamide (the compound of Example 65);
(3a*S**,4*S**,9b*S**)-*N*-[2-(8-cyano-2,3,3a,4,5,9b-hexahydro-furo[3,2-*c*]quinolin-4-yl)-2-methyl-propyl]-4-trifluoromethoxybenzamide (the compound of Example 113);
(3a*S**,4*S**,9b*S**)-*N*-[2-(8-cyano-2,3,3a,4,5,9b-hexahydro-furo[3,2-*c*]quinolin-4-yl)-2-methyl-propyl]-4-ethoxybenzamide (the compound of Example 114);
(3a*R**,4*S**,9b*S**)-6-acetylamino-*N*-[2-(8-cyano-3a,4,5,9b-tetrahydro-3H-cyclopenta[c]quinolin-4-yl)-2-methyl-propyl]nicotinamide (the compound of Example 108);
(3a*S**,4*R**,9b*S**)-4-acetylamino-*N*-[2-(8-cyano-2,3,3a,4,5,9b-hexahydro-furo[3,2-*c*]quinolin-4-yl)propyl]benzamide (the compound of Example 129).

The salts of the compounds of the present invention represented by the formula (I) are not limited to any particular types, as long as they are pharmacologically acceptable. Their examples include: hydrohalogenic acid salts, such as hydrofluorides, hydrochlorides, hydrobromides and hydroiodides; inorganic acid salts, such as nitrates, perchlorates, sulfates, phosphates and carbonates; lower alkylsulfonic acid salts, such as methanesulfonates, trifluoromethanesulfonates and ethanesulfonates; arylsulfonic acid salts, such as benzenesulfonates and *p*-toluenesulfonates; acetates, fumarates; amino acid salts, such as glycine salts, alanine salts, glutamates, and aspartates; and alkali metal salts, such as sodium salts and potassium salts. Solvates of the compounds of the present invention are also included in the present invention. Examples of the solvates are solvates with solvents, such as acetone, 2-butanol, 2-propanol, ethanol, ethyl acetate, tetrahydrofuran, and diethyl ether.

The tetrahydroquinoline derivatives of the present invention can be produced by the following methods.

### [Production Method 1]

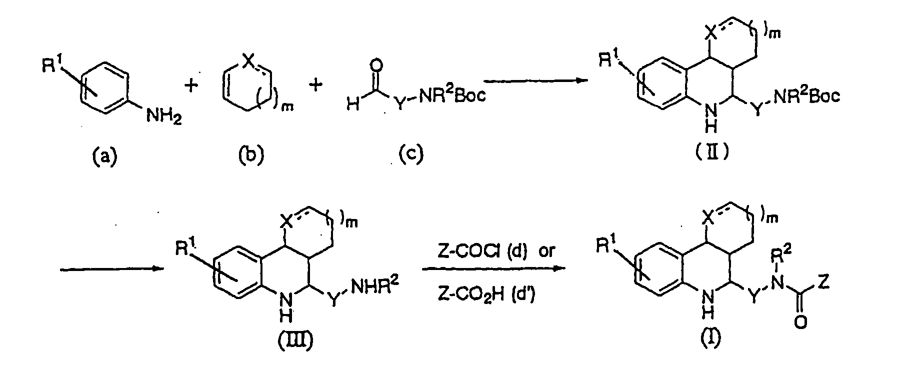

(where all symbols are as defined above, and Boc represents a *tert*-butoxycarbonyl group.)

The compound of the present invention, expressed by the formula (I), can be produced by the reactions involved in the following steps 1, 2, and 3.
(Step 1) In this step, the compounds represented by the formulas (a), (b) and (c) are subjected to reaction in an inert solvent in the presence or absence of an acid, thereby producing the compound represented by the formula (II).

The compounds represented by the formulas (a), (b) and (c) can be obtained as commercially available reagents, or by easy derivation therefrom by routine chemical reactions.

The present reaction will be described concretely. Any type of acids, organic or inorganic, are preferred. For example, acetic acid, trifluoroacetic acid, *p*-toluenesulfonic acid, hydrochloric acid, sulfuric acid, tin tetrachloride, titanium tetrachloride, boron trifluoride diethyl etherate, diethylaluminum chloride, or ethylaluminum dichloride may be used. The acid is preferably used in an amount ranging from a catalytic amount to 10 equivalents with respect to the compound represented by the formula (a). The reaction solvent is not limited in any particular way as long as it does not markedly impede the present reaction. The preferred reaction solvent is dichloromethane, chloroform, 1,2-dichloroethane, hexane, benzene, toluene, dioxane, tetrahydrofuran, acetonitrile, methanol, ethanol, water or a mixture of these solvents. The reaction temperature is preferably -20 to 100°C, and the reaction time is preferably 5 minutes to 48 hours.

### (Step 2)

In this step, the compound represented by the formula (II) may be deprotected by treatment with an acid to produce the compound represented by the formula (III).

The present reaction will be described concretely. Any type of acids, organic or inorganic, are preferred. For example, acetic acid, trifluoroacetic acid, *p*-toluenesulfonic acid, hydrochloric acid or sulfuric acid may be used. The acid is preferably used in an amount ranging from 1 to 50 equivalents with respect to the compound represented by the formula (II). The reaction solvent is not limited to any particular type, as long as it does not markedly impede the present reaction. The preferred reaction solvent is dichloromethane, chloroform, 1,2-dichloroethane, hexane, benzene, toluene, dioxane, tetrahydrofuran, acetonitrile, methanol, ethanol, water or a mixture of these solvents. The reaction temperature is preferably 0 to 100°C, and the reaction time is preferably 30 minutes to 24 hours.

### (Step 3)

In this step, the compound represented by the formula (III) may be reacted with the compound represented by the formula (d) or the formula (d') either without a solvent or in an inert solvent in the presence or absence of a base to form an amide bond, thereby producing the compound represented by the formula (I).

The reactions for forming an amide bond are known and may be exemplified by a method involving the use of an acid halide and a method involving the use of a condensing agent.

These reactions will be described concretely. The method involving the use of an acid halide is such that an acid halide represented by the formula (d) is reacted with the compound represented by the formula (III) either without a solvent or in an inert solvent in the presence or absence of a base to form an amide. The base is preferably a tertiary amine, and its examples are triethylamine and pyridine. The acid halide represented by the formula (d) is preferably used in an amount ranging from 1 to 10 equivalents with respect to the compound represented by the formula (III). The base is preferably used in an amount ranging from 1 equivalent to a large excess with respect to the acid halide. The reaction solvent is not limited in any particular way, as long as it does not markedly impede the present reaction. Preferred reaction solvents are, for example, dichloromethane, chloroform, 1,2-dichloroethane, 1,1,2,2-tetrachloroethane, toluene and pyridine. The reaction temperature is preferably 0 to 80°C, and the reaction time is preferably 30 minutes to 24 hours.

The method involving the use of a condensing agent is such that the compound represented by the formula (III) is reacted with the compound represented by the formula (d') using a condensing agent such as 2-chloro-1,3-dimethylimidazolinium chloride, dicyclohexylcarbodiimide or 1-ethyl-3-(3-dimethylaminopropyl)carbodiimide either without a solvent or in an inert solvent in the presence or absence of a base. The condensing agent is preferably used in an amount ranging from 1 to 2 equivalents with respect to the compound represented by the formula (d'). The base is preferably a tertiary amine, and its examples are 4-methylmorpholine, triethylamine and pyridine. The base is preferably used in an amount ranging from 1 equivalent to a large excess with respect to the compound represented by the formula (d'). The reaction solvent is not limited in any particular way, as long as it does not markedly impede the present reaction. Preferred reaction solvents are, for example, *N*,*N*-dimethylformamide, dimethyl sulfoxide, tetrahydrofuran, dioxane, dichloromethane, chloroform and 1,2-dichloroethane. The reaction temperature is preferably 0 to 150°C, and the reaction time is preferably 1 to 48 hours.

The compounds of the present invention, which are produced by the above-described methods, are isolated and purified as free compounds, their salts, various solvates thereof such as hydrates or ethanolates, or crystalline polymorphic substances. The pharmacologically acceptable salts of the compounds according to the present invention can be produced by the usual salt-forming reaction. The isolation and purification are performed by applying chemical operations, such as extractive fractionation, crystallization, and various fractional chromatographic techniques. The stereochemically pure optical isomers can be synthesized by using suitable starting compounds, or by optical resolution of racemic compounds.

The tetrahydroquinoline derivatives or pharmacologically acceptable salts thereof according to the present invention have excellent AR agonism. These substances can be used as active ingredients to form pharmaceuticals or AR agonists and can widely be applied in the prevention or treatment of various AR-related diseases.

The AR agonism is expected to provide treatment of the following diseases.

Diseases which can potentially be treated by protein anabolic action may include the following examples: primary osteoporosis (senile, postmenopausal and juvenile osteoporosis) and secondary osteoporosis [osteoporosis ascribed to hyperthyroidism, Cushing syndrome (due to steroid treatment), acromegaly, hypogonadism, dysosteogenesis, hypophosphatasemia or diabetes, or disuse osteoporosis], in view of the potent action on bone tissue; wasting diseases that derive postoperatively or from malignant tumor, trauma, chronic renal disease, burns or AIDS infection, in view of the potent action on muscular tissues; hematopoietic dysfunction and diseases related thereto, such as aplastic anemia, hemolytic anemia, sickle cell anemia, idiopathic thrombocytopenic purpura, myelofibrosis and renal anemia, in view of the erythropoiesis promoting action.

Diseases which can be potentially cured on account of sexual action may include, for example, male hypogonadism, male sexual dysfunction (impotence, male dysspermatogenic sterility), abnormal sex differentiation (male hermaphroditism), male delayed puberty, cancer in female genital organ (including the pain which accompanies cancer), breast cancer, mastopathy, endometriosis and female sexual dysfunction.

The pharmaceuticals of the present invention can be applied widely to these AR-related diseases, and may also be applied to diseases which are not mentioned here, if they require modulation of the AR function either at present or in the future.

The pharmaceuticals of the present invention can be prepared as pharmaceutical compositions by mixing the active ingredient with those carriers, excipients (both may be organic or inorganic in either a solid or liquid form), auxiliary substances, stabilizers, wetting agents, emulsifying agents, buffers and various other pharmacologically acceptable additives which are employed in the usual pharmaceutical formulation procedures, and they can be formulated in various dosage forms that may be chosen as appropriate for a particular route of administration.

The pharmaceuticals of the present invention can be administered orally or parenterally, and may be of the systemic administration type or local administration type.

Their dosage forms are not limited in any particular way and may include tablets, capsules, sugar-coated tablets, granules, subtilized granules, inhalants, suppositories, liquids and solutions, syrups, dry syrups, suspensions, emulsions, lotions, ointments, patches, sprays, gels, nasal drops, eye drops, and injections.

The dose for administering the pharmaceuticals of the present invention to humans may be determined appropriately by various conditions, such as the purpose of treatment or prevention, the patient's sex, body weight, age, the state of his or her health, the type and severity of the disease, dosage form, the route of administration, and the duration of administration. The daily dose of the tetrahydroquinoline derivatives of the present invention generally ranges from 0.01 to 100 mg/kg.

The pharmaceuticals of the present invention may also be used in the treatment of androgen receptor-mediated diseases in warm-blooded animals, such as domestic animals, pets, bred animals, or wild animals. The dosage forms and doses in this case can be determined by reference to the dosage forms and doses for humans.

### Examples

The compounds of the present invention and the methods for their production will be described in further detail by working examples. However, the present invention is not to be interpreted in a limited way because of these descriptions.

¹H-NMR spectra were recorded on JNM-EX270 Spectrometer (270 MHz, JEOL Ltd.). Chemical shifts (δ) are expressed in ppm downfield from tetramethylsilane (TMS) as an internal standard.

In the structural formulas and tables set forth below, Me represents a methyl group, Et an ethyl group, Pr a propyl group, Bu a butyl group, Ph a phenyl group, Bn a benzyl group, and Ac an acetyl group.

[Example 1] Production of (3a*R**,4*S**,9b*S**)-2-methoxy-*N*-[2-methyl-2-(8-nitro-3a,4,5,9b-tetrahydro-3*H*-cyclopenta[*c*]quinolin-4-yl)propyl]-1-acetamide 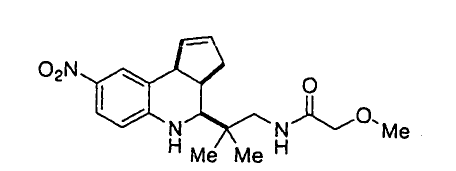 (for the sake of convenience, only one side of the absolute configuration is indicated in the chemical structural formula and the other side is omitted, as in the following chemical structural formulas.)

### (1) (3aR*,4S*,9bS*)-[2-methyl-2-(8-nitro-3a,4,5,9b-tetrahydro-3H-cyclopenta[c]quinolin-4-yl)propyl]carbamate tert-butyl ester

4-Nitroaniline (16.2 g), cyclopentadiene (11.7 ml) and (2,2-dimethyl-3-oxo-propyl)carbamate *tert*-butyl ester (23.6 g) were dissolved in acetonitrile (120 ml) and trifluoroacetic acid (4.5 ml) was added at 0°C. After stirring overnight at room temperature, the precipitating crystal was recovered by filtration to give the title compound (16.8 g). Its physical properties are shown below.

¹H-NMR(CDCl₃) δ: 0.99(3H, s), 1.03(3H, s), 1.34(9H, s), 2.31-2.23(1H, m), 2.54-2.44(1H, m), 2.84-2.92(2H, m), 3.34-3.46 (2H, m), 3.98(1H, d, J= 8.6Hz), 4.70(1H, m), 4.84(1H, brs), 5.74-5.77(1H, m), 5.94-5.98(1H, m), 6.58(1H, d, J= 8.6Hz), 7.82-7.86(2H, m).

### (2) (3aR*,4S*,9bS*)-2-methyl-2-(8-nitro-3a,4,5,9b-tetrahydro-3H-cyclopenta[c]quinolin-4-yl)propylamine

The compound obtained in (1) in an amount of 16.8 g was dissolved in tetrahydrofuran (100 ml) and 4N HCl-dioxane solution (50 ml) was added. After stirring at 50°C for 3 hours, the solvent was distilled off under reduced pressure. The residue was washed with ether to give the title compound (15.2 g). Its physical properties are shown below.

¹H-NMR(CDCl₃) δ: 1.17(3H, s), 1.24(3H, s), 2.22-2.27 (1H, m), 2.37-2.47(1H, m), 3.47(1H, dd, J= 7.3, 14.2Hz), 3.52(1H, brs), 4.00(1H, d, J= 8.6Hz), 5.72-5.74(1H, m), 5.95-5.97(1H, m), 6.38(1H, brs), 6.90(1H, d, J= 9.9Hz), 7.45(1H, s), 7.77-7.81 (2H, m), 8.01(1H, brs). (3) (3a*R**,4*S**,9b*S**)-2-methoxy-*N*-[2-methyl-2-(8-nitro-3a,4,5,9b-tetrahydro-3*H*-cyclopenta[*c*]quinolin-4-yl)propyl]-1-acetamide

The compound obtained in (2) in an amount of 15.2 g and triethylamine (12 ml) were dissolved in N,N-dimethylformamide (50 ml) and methoxyacetyl chloride (4.8 ml) was added. After 5 hours of stirring at room temperature, water and ethyl acetate were added. The ethyl acetate layer was washed with water and dried over anhydrous sodium sulfate, and then the solvent was distilled off under reduced pressure. The residue was purified by silica gel column chromatography (elution solvent; hexane:ethyl acetate = 1:3) to yield the title compound (12.6 g). Its physical properties are shown below.

¹H-NMR(CDCl₃) δ: 1.04(3H, s), 1.09(3H, s). 2.28(1H, dd, J= 8.2, 15.5Hz), 2.43-2.53(1H, m), 2.86(1H, dd, J= 5.6, 14.5Hz), 3.29(1H, d, J= 2.3Hz), 3.41(3H, s), 3.66(1H, dd, J= 8.6, 14.5Hz), 3.87(3H, s), 3.96(1H, d, J= 8.6Hz), 4.89(1H, brs), 5.77(1H, brs), 5.97-5.99(1H, m), 6.60(1H, d, J= 9.6Hz), 6.75(1H, brs), 7.83-7.87(2H, m).

Compounds shown in Examples 2 to 25 were produced in the same manner as in Example 1. The physical properties of the resulting compounds are shown in Tables 1 to 4.

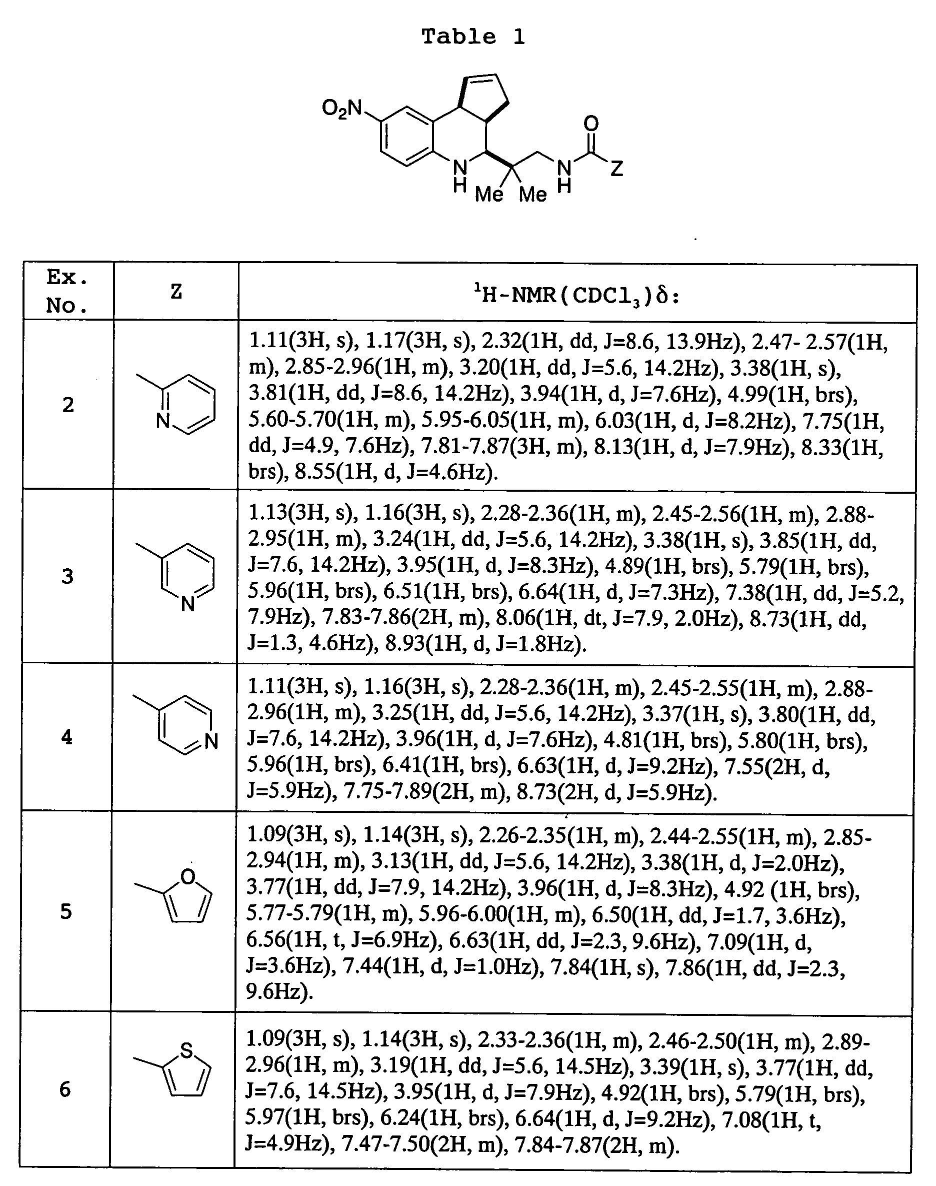

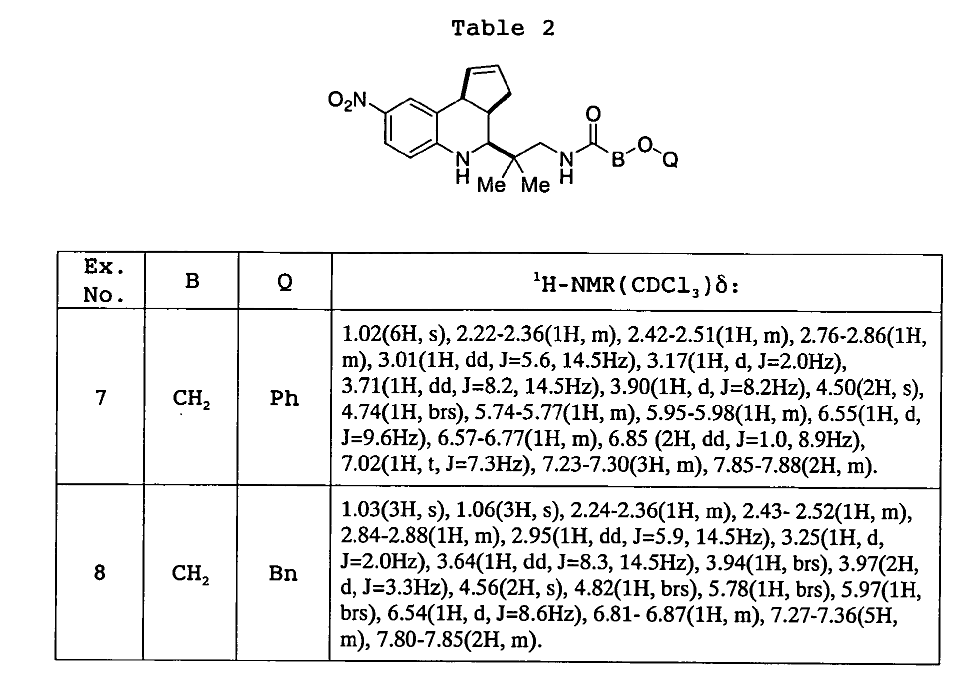

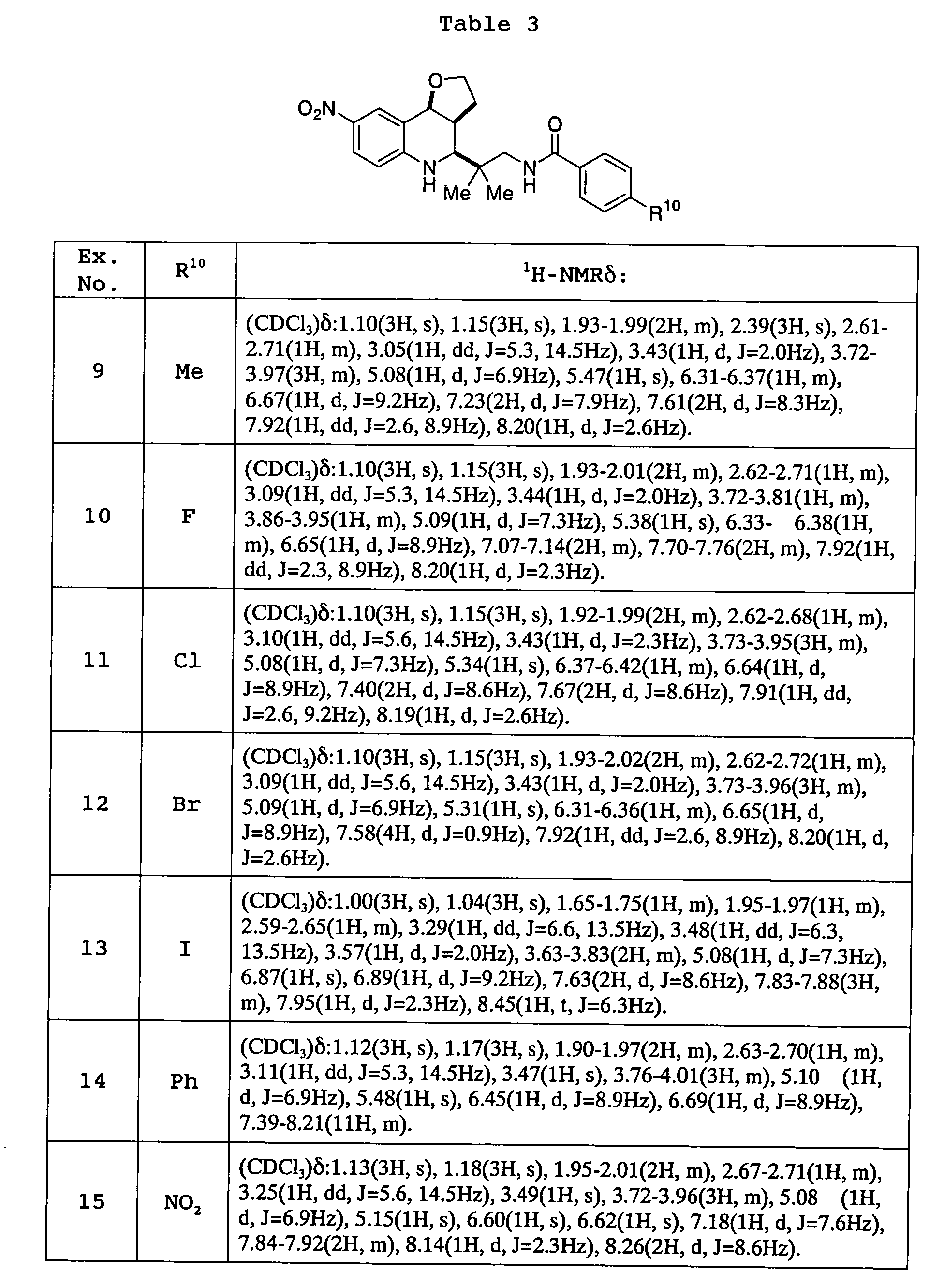

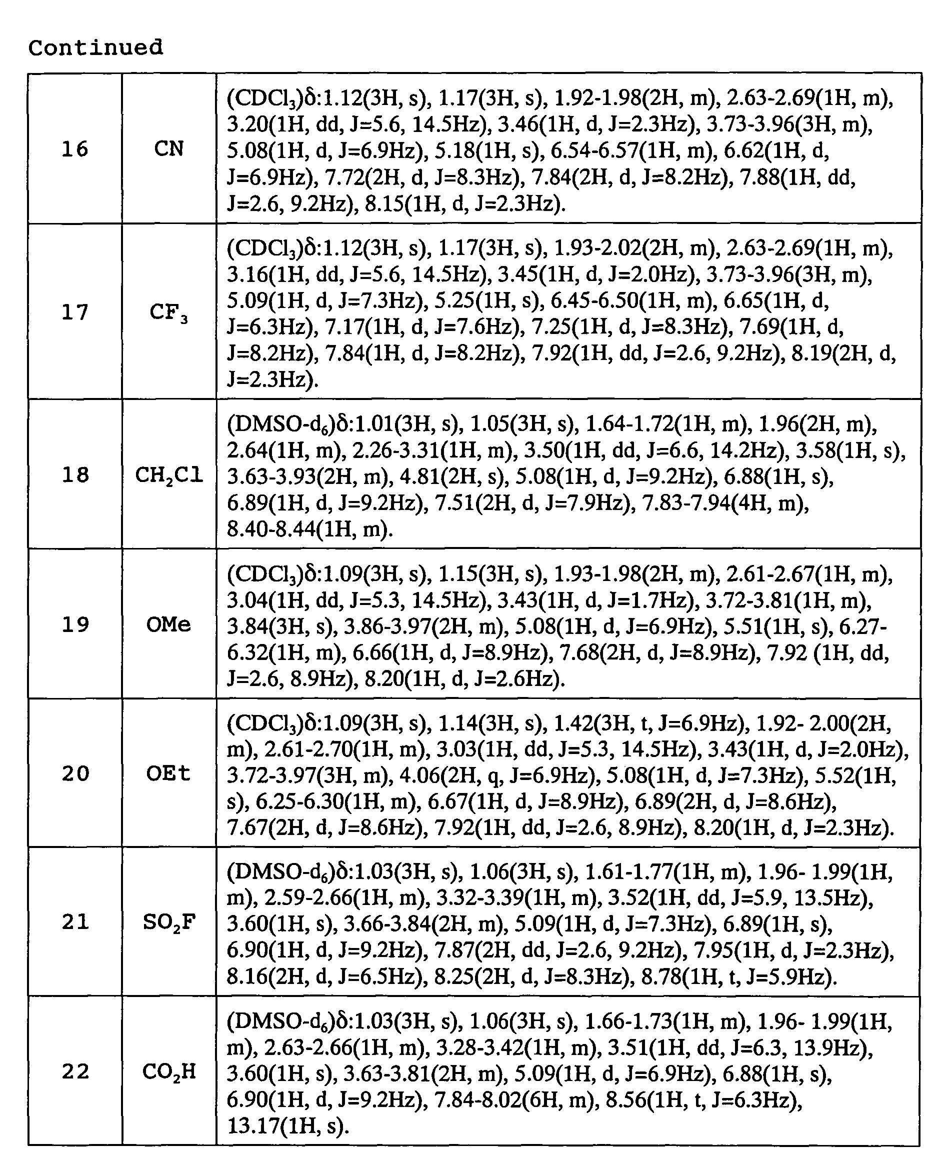

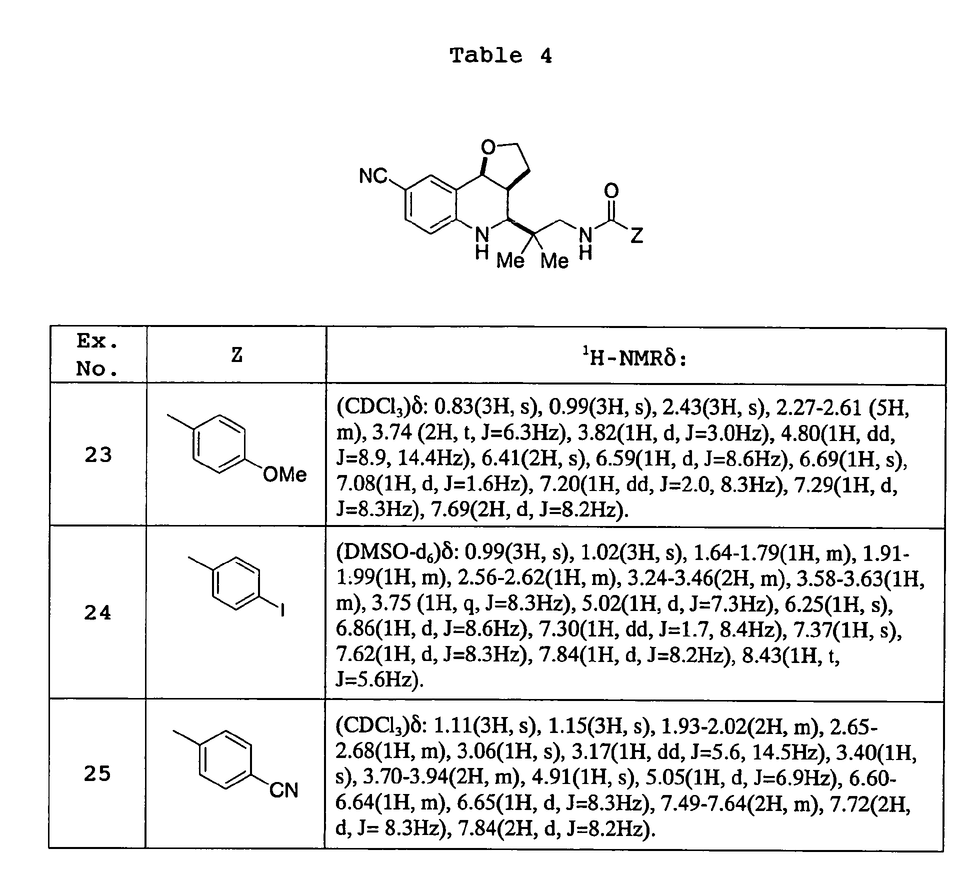

[Example 26] Production of (3a*R**,4*S**,9b*S**)-2-ethoxy-*N*-[2-methyl-2-(8-nitro-3a,4,5,9b-tetrahydro-3*H*-cyclopenta[*c*]quinolin-4-yl)propyl]-1-acetamide

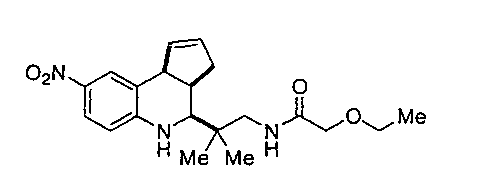

A hundred milligrams of the (3a*R**,4*S**,9b*S**)-2-methyl-2-(8-nitro-3a,4,5,9b-tetrahydro-3*H*-cyclopenta[*c*]quinolin-4-yl)propylamine obtained in Example 1-(2) and ethoxyacetic acid (0.04 ml) were dissolved in N,N-dimethylformamide (3 ml), and 4-methylmorpholine (0.1 ml), 1-hydroxybenzotriazole (60.0 mg) and 1-ethyl-3-(3-dimethylaminopropyl)carbodiimide hydrochloride (90.0 mg) were added. After 2 hours of stirring at room temperature, water and ethyl acetate were added. The ethyl acetate layer was washed with water and dried over anhydrous sodium sulfate, and then the solvent was distilled off under reduced pressure. The residue was purified by silica gel column chromatography (elution solvent; hexane:ethyl acetate = 1:2) to yield the title compound (80.0 mg). Its physical properties are shown below.

¹H-NMR(CDCl₃) δ: 1.04(3H, s), 1.09(3H, s), 1.23(3H, t, J= 6.9Hz), 2.25-2.33(1H, m), 2.43-2.53(1H, m), 2.82-2.89(1H, m), 2.98(1H, dd, J= 5.5, 14.2Hz), 3.29(3H, s), 3.56(1H, q, J= 6.9Hz), 3.66(1H, dd, J= 8.3, 14.2Hz), 3.91(3H, s), 3.96(1H, d, J= 6.3Hz), 4.90(1H, brs), 5.78(1H, brs), 5.97(1H, brs), 6.60(1H, d, J= 9.6Hz), 6.81(1H, brs), 7.83-7.86(2H, m).

Compounds shown in Examples 27 to 131 were produced in the same manner as in Example 26. The physical properties of the resulting compounds are shown in Tables 5 to 14.

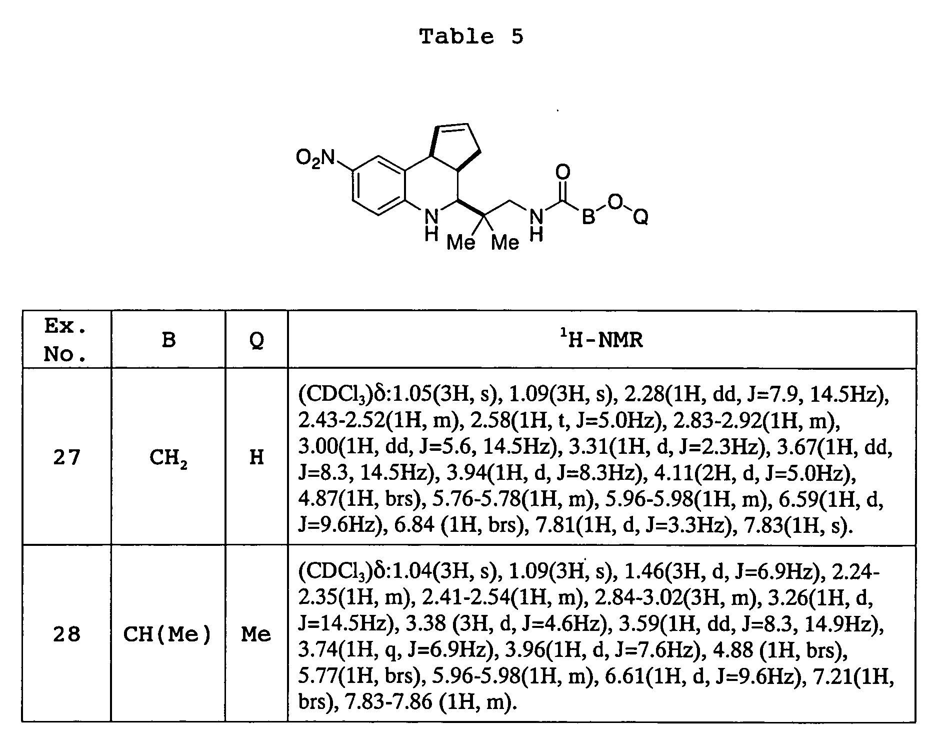

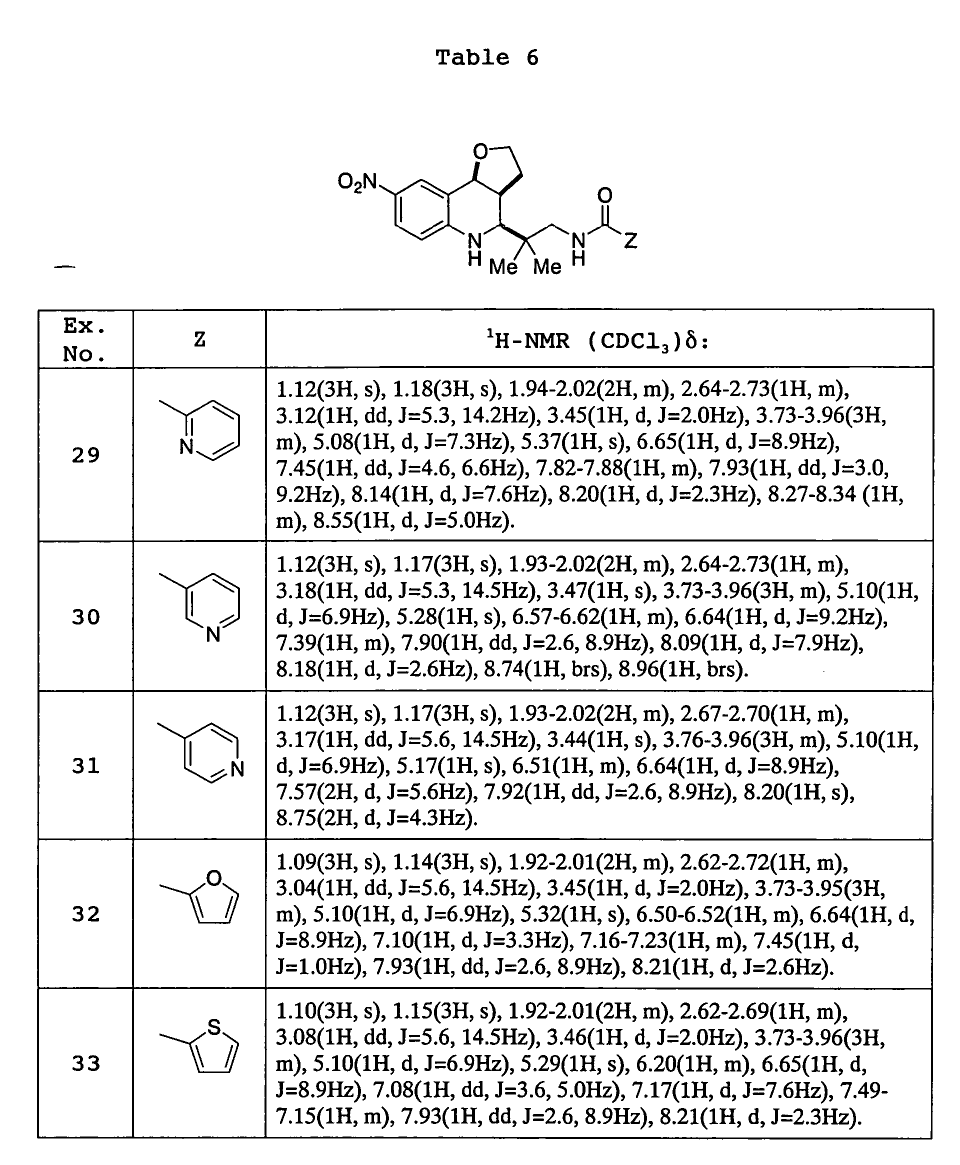

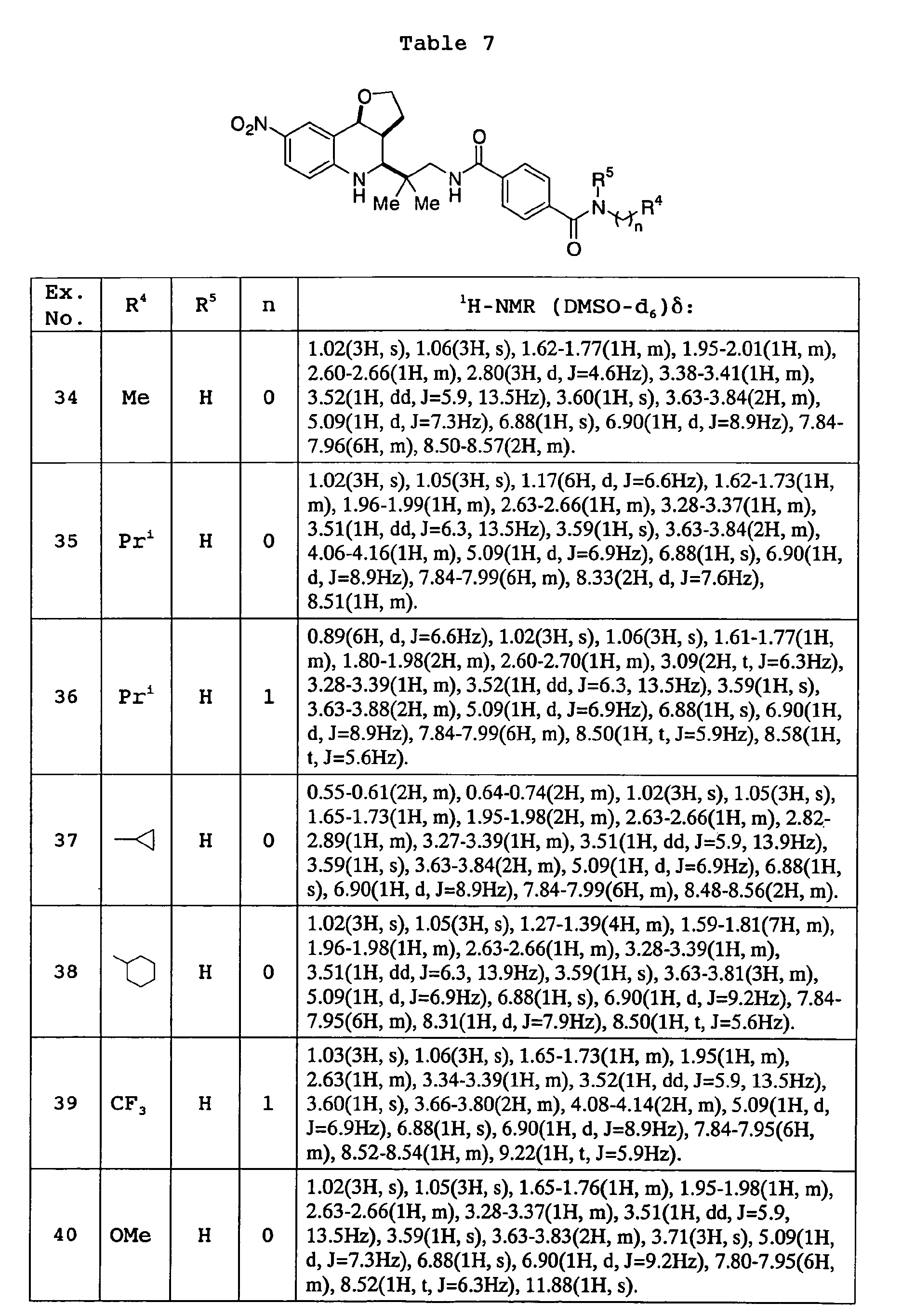

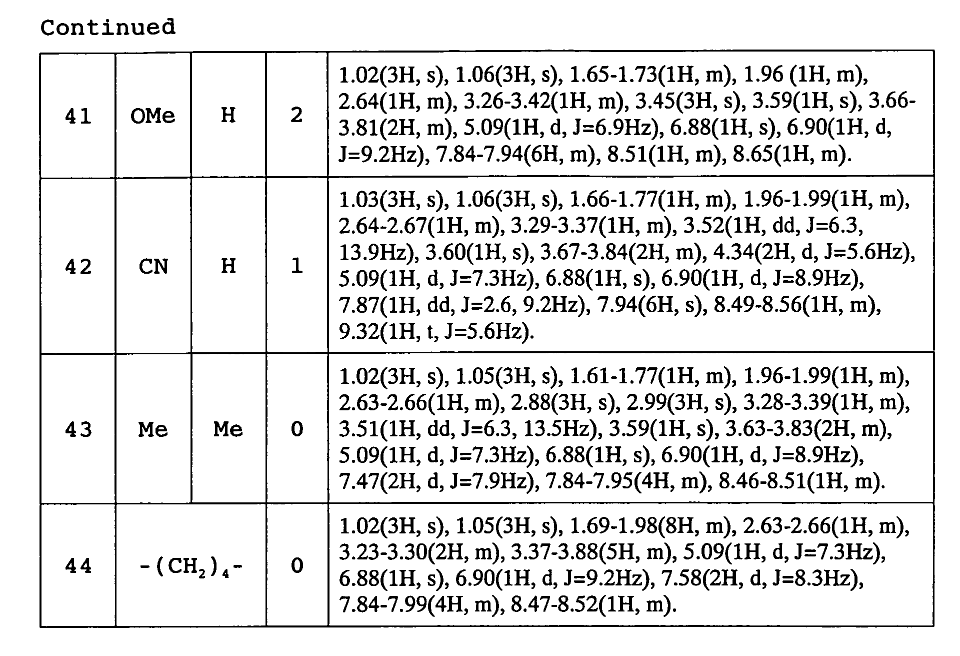

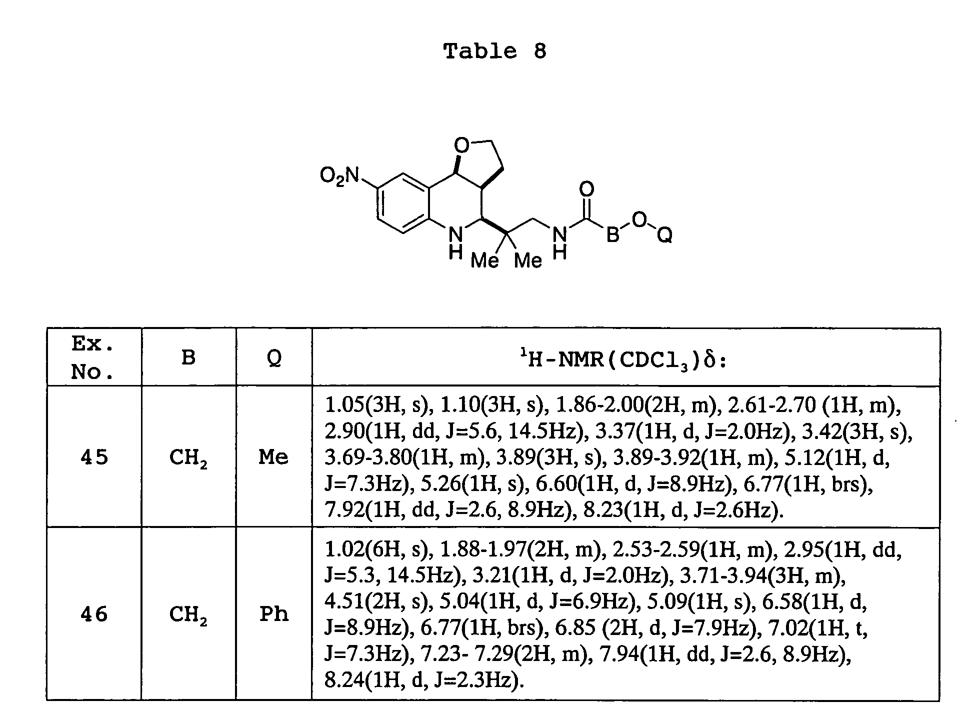

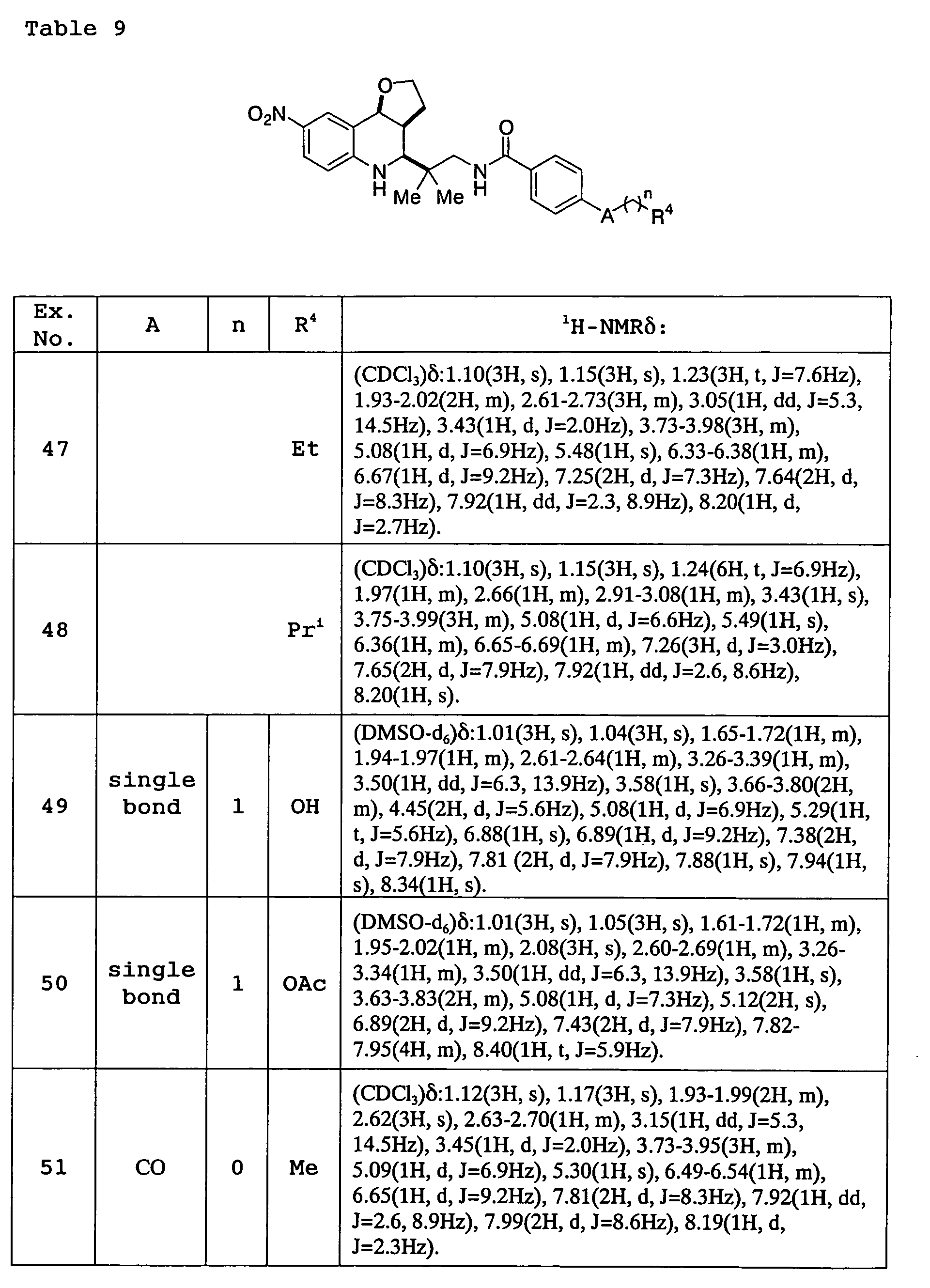

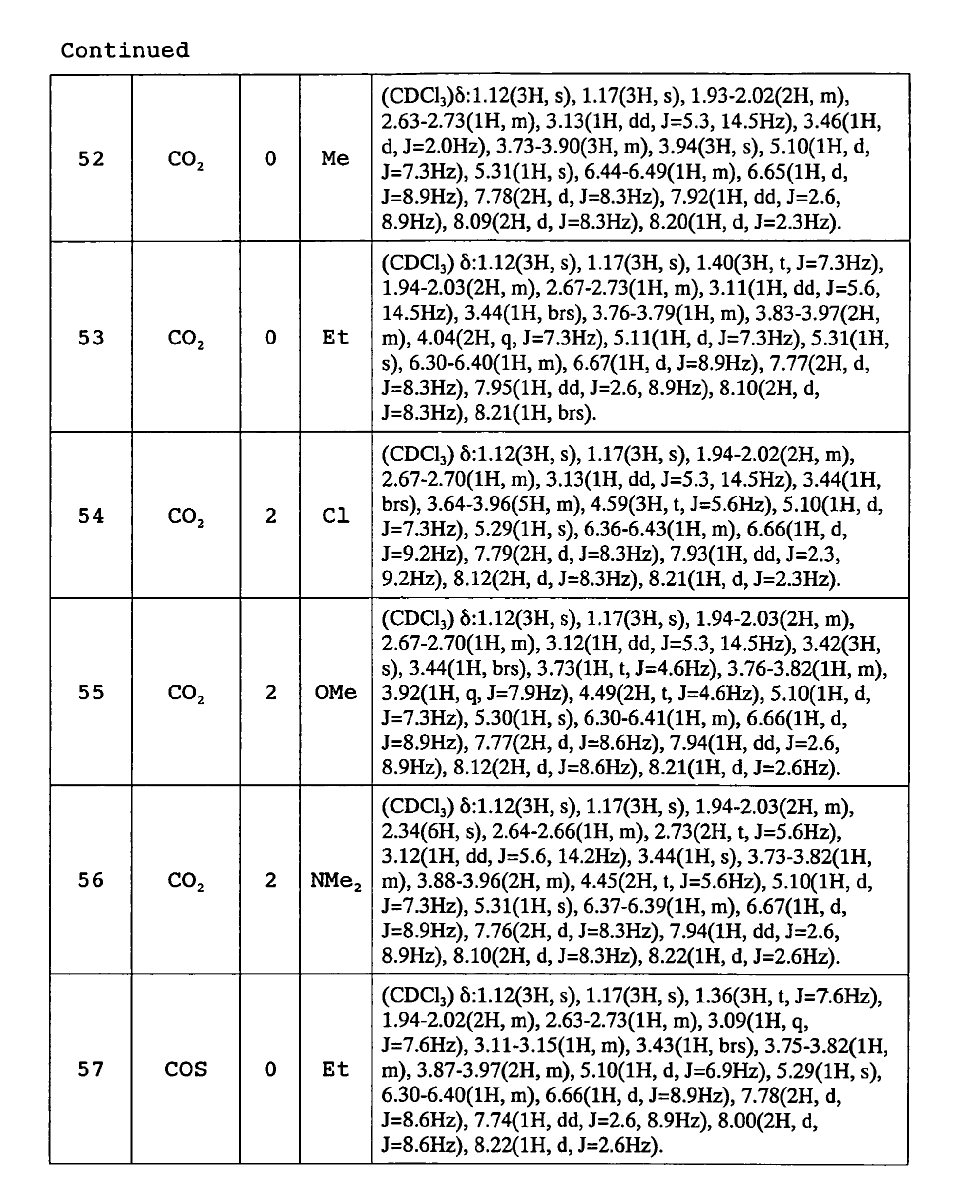

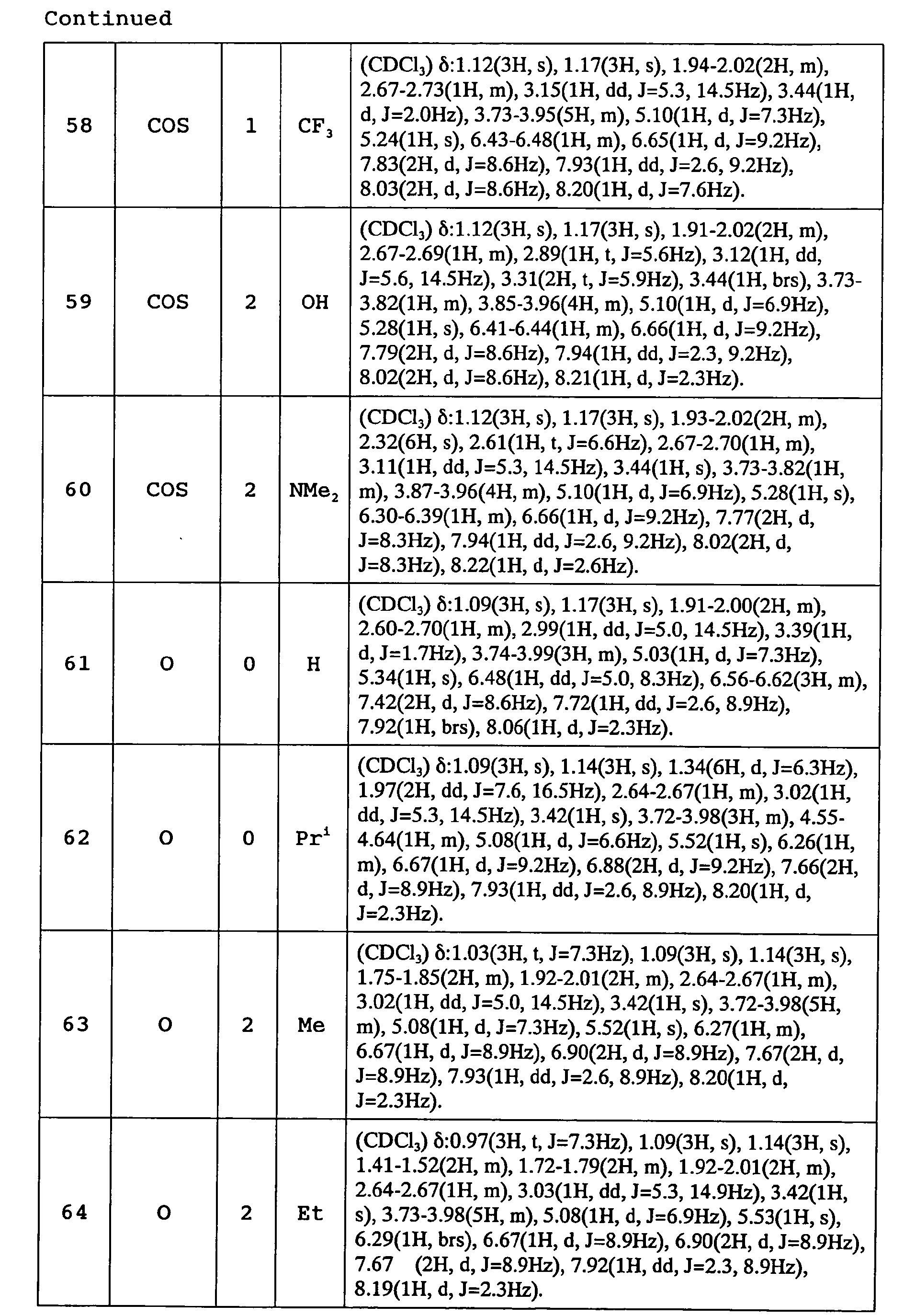

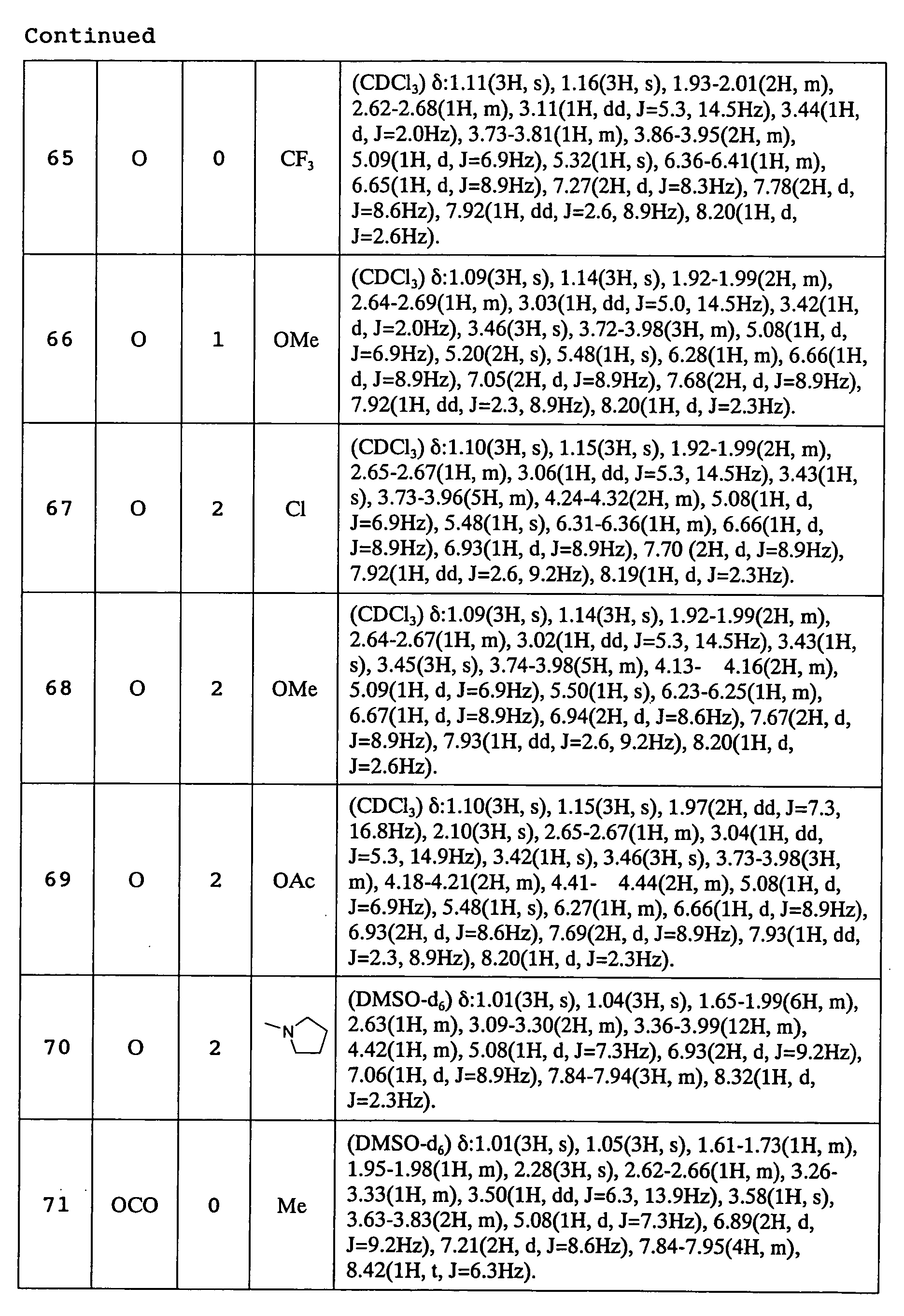

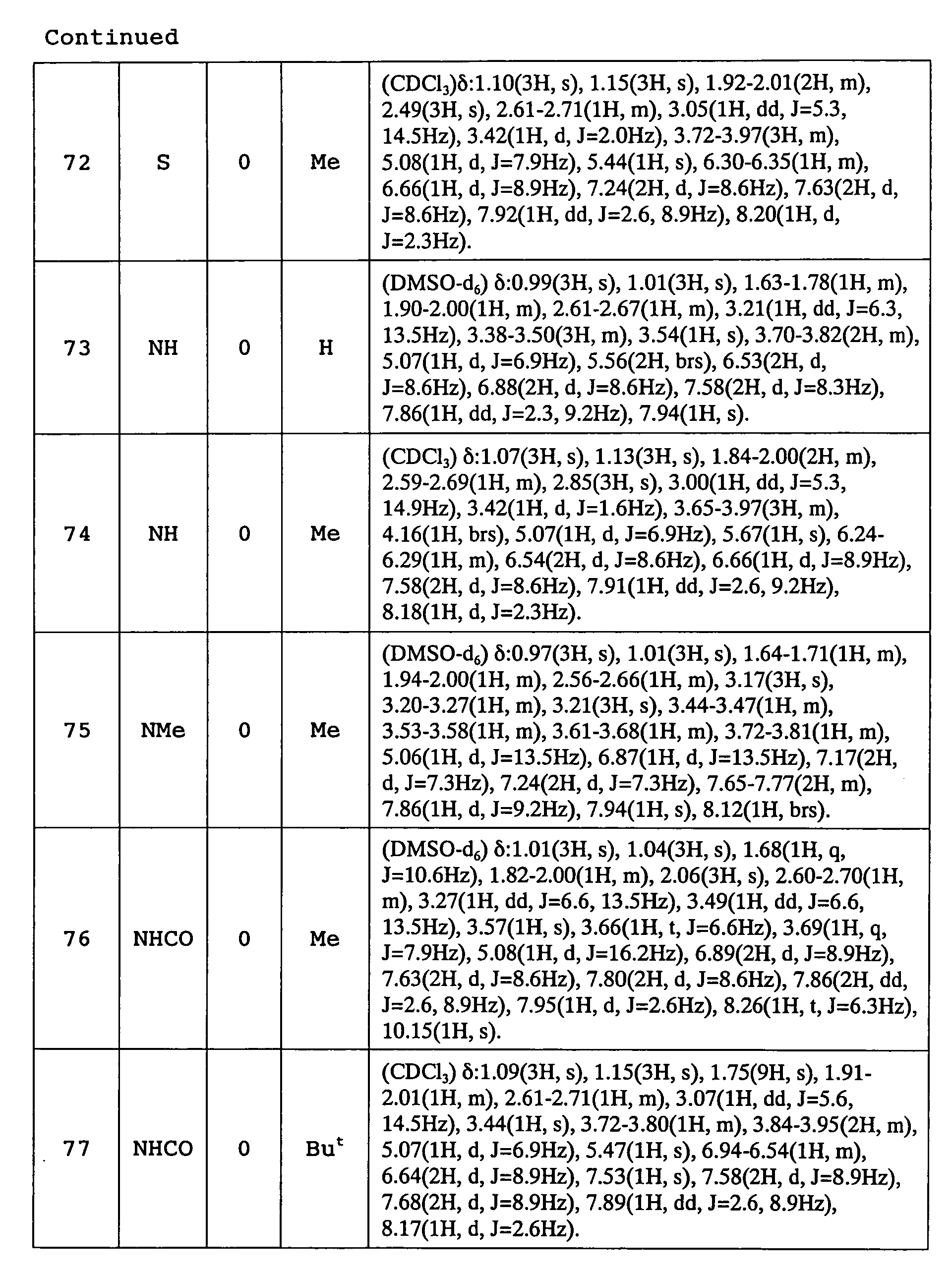

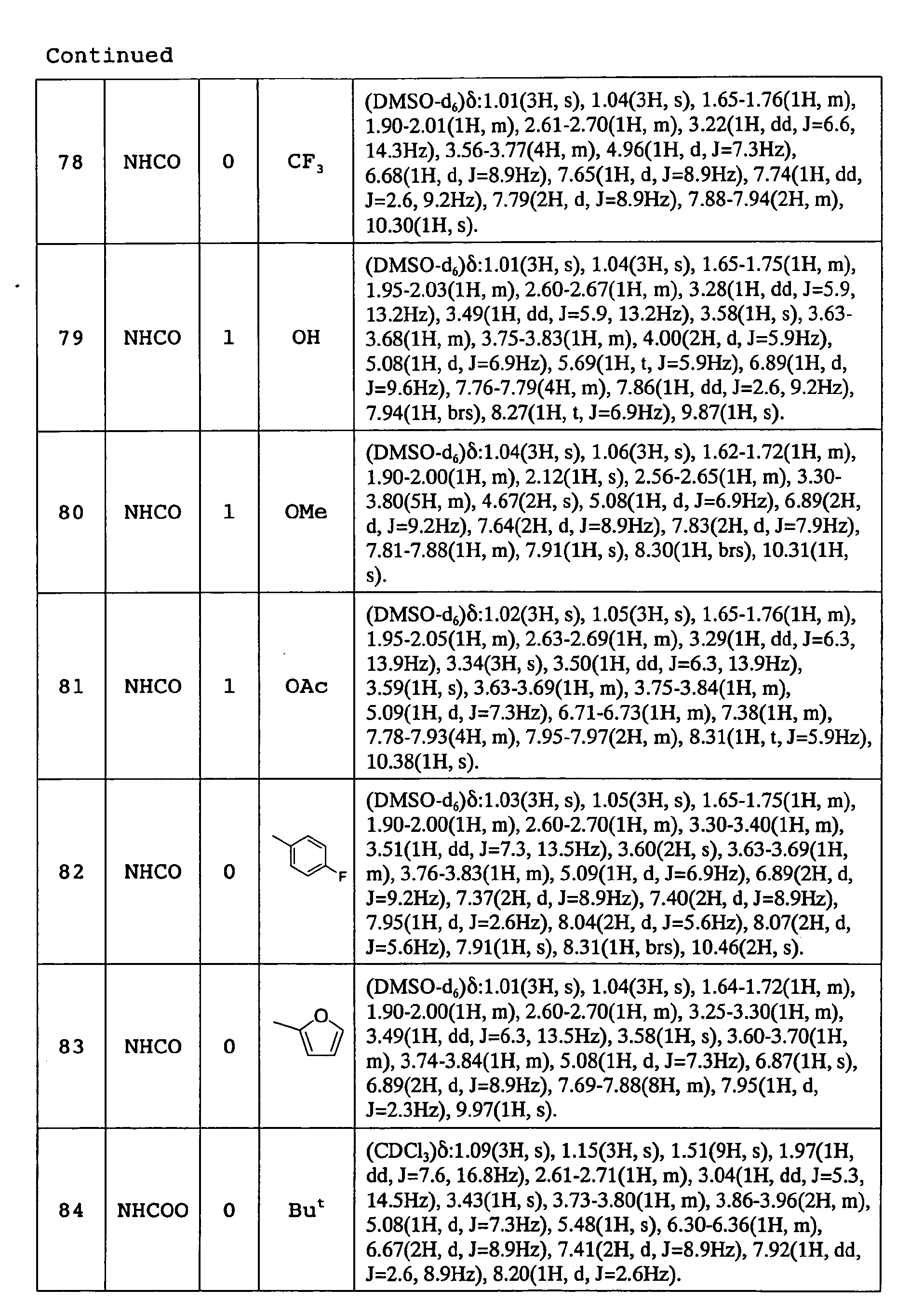

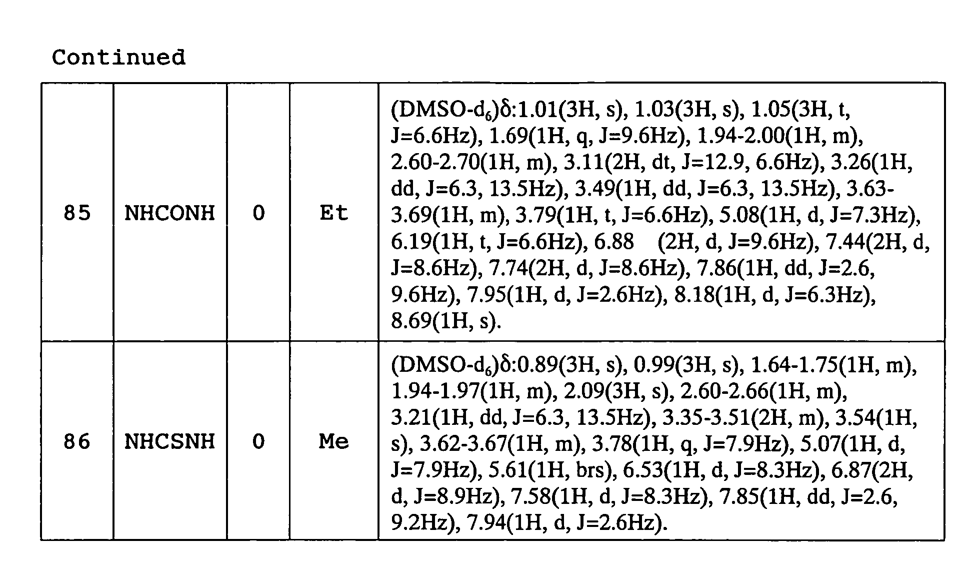

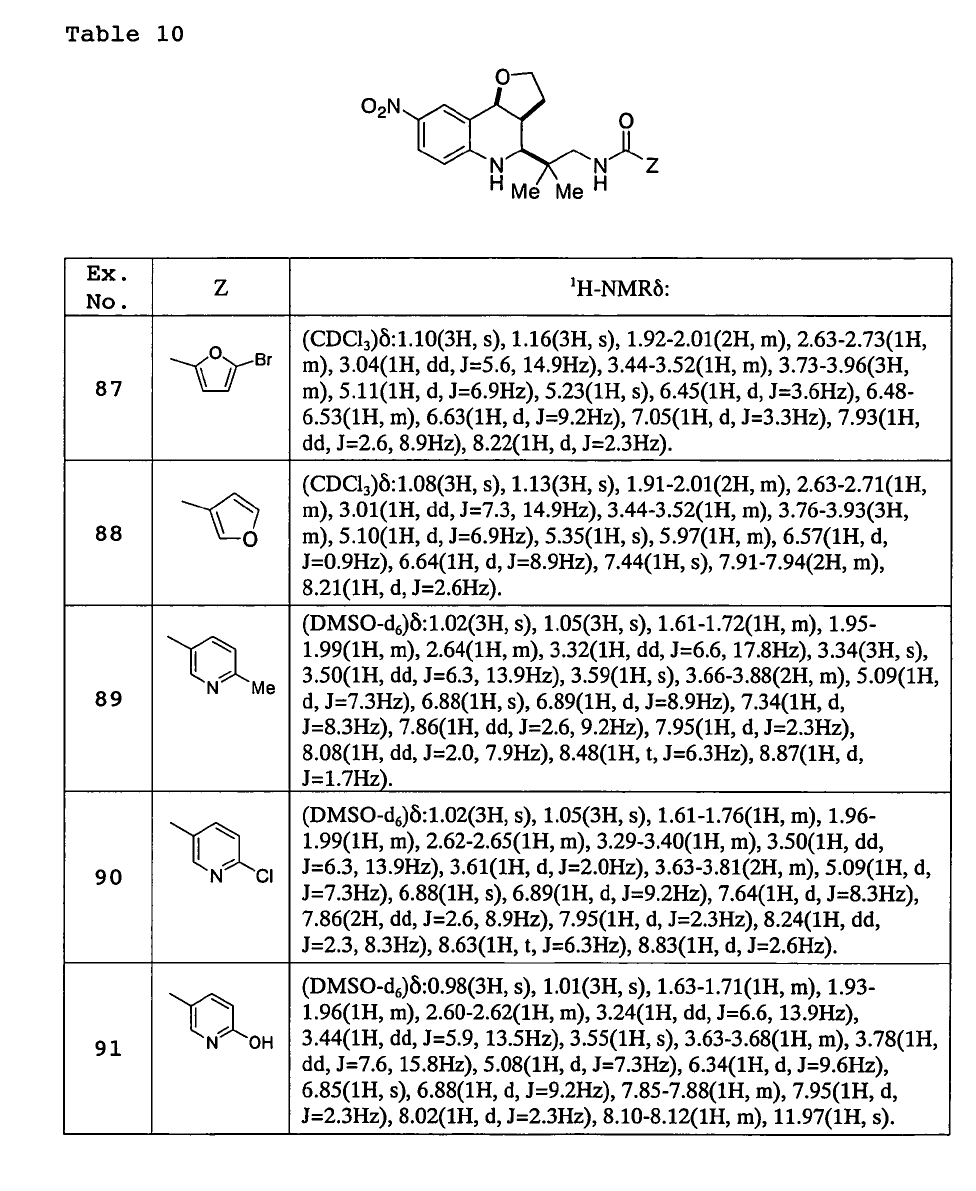

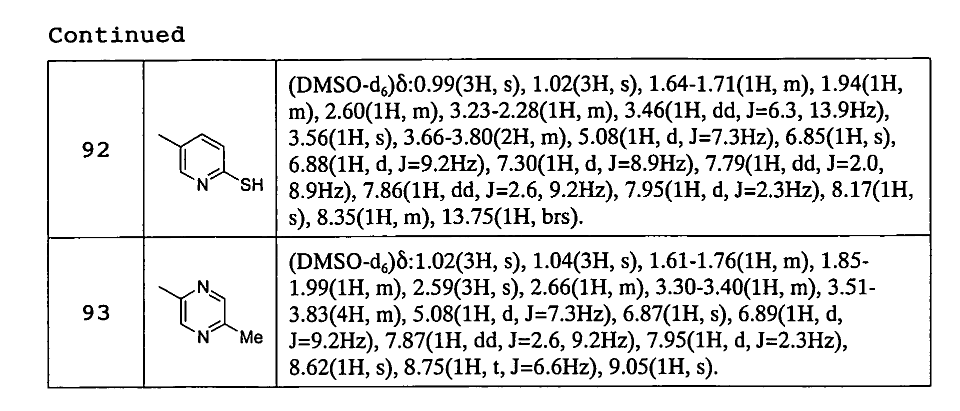

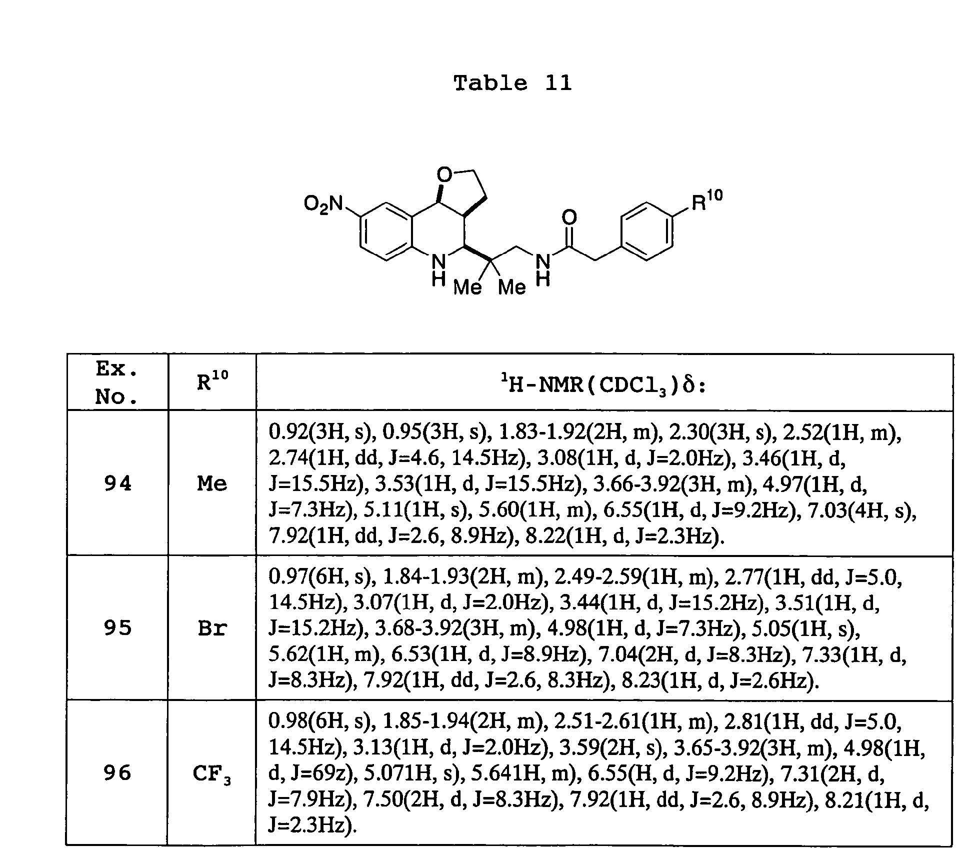

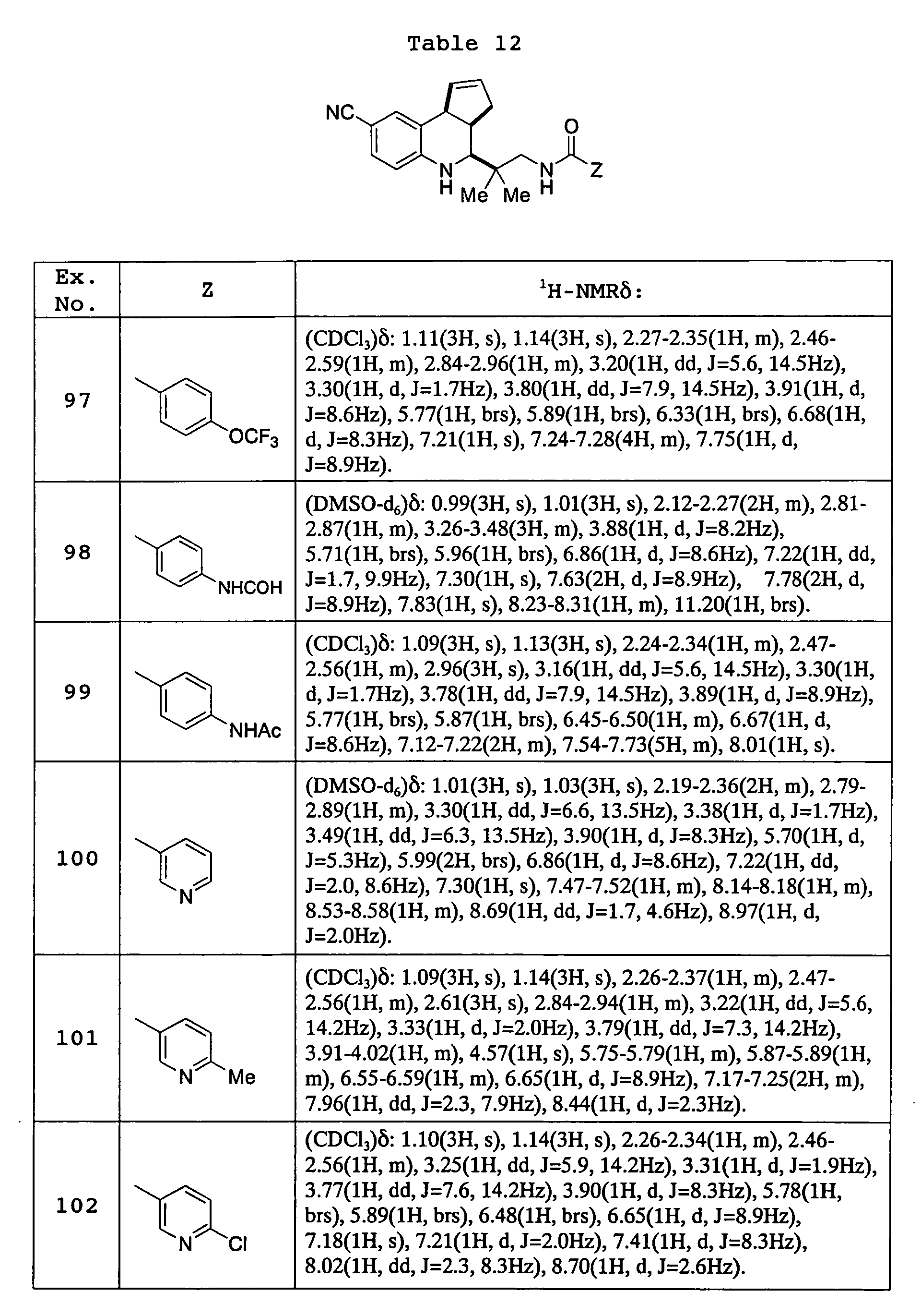

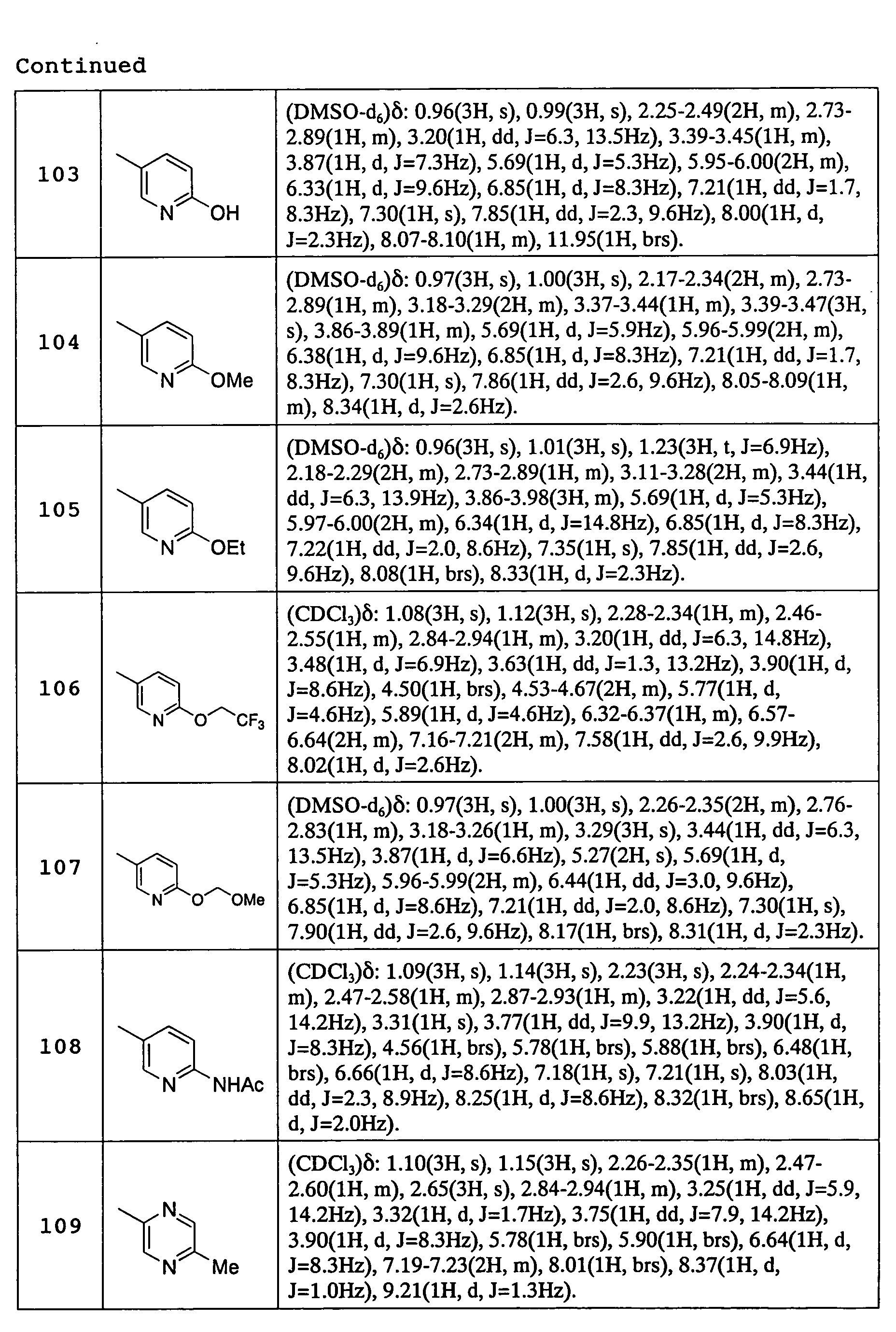

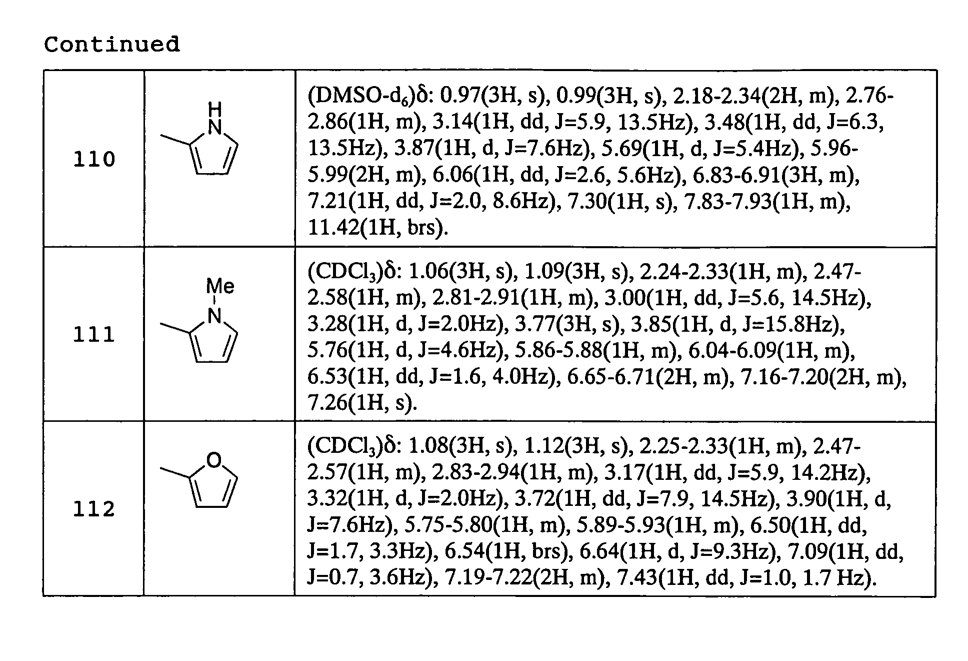

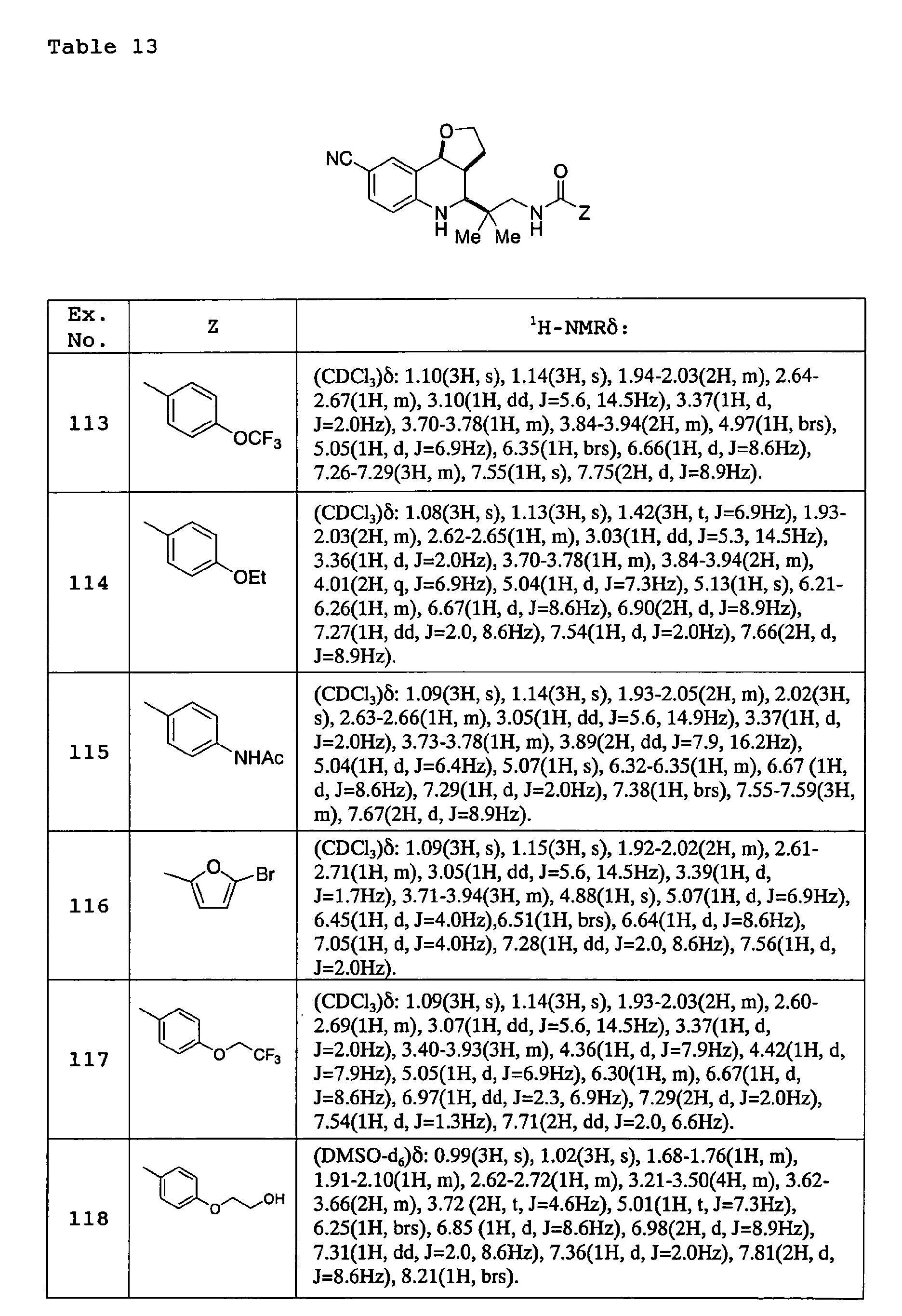

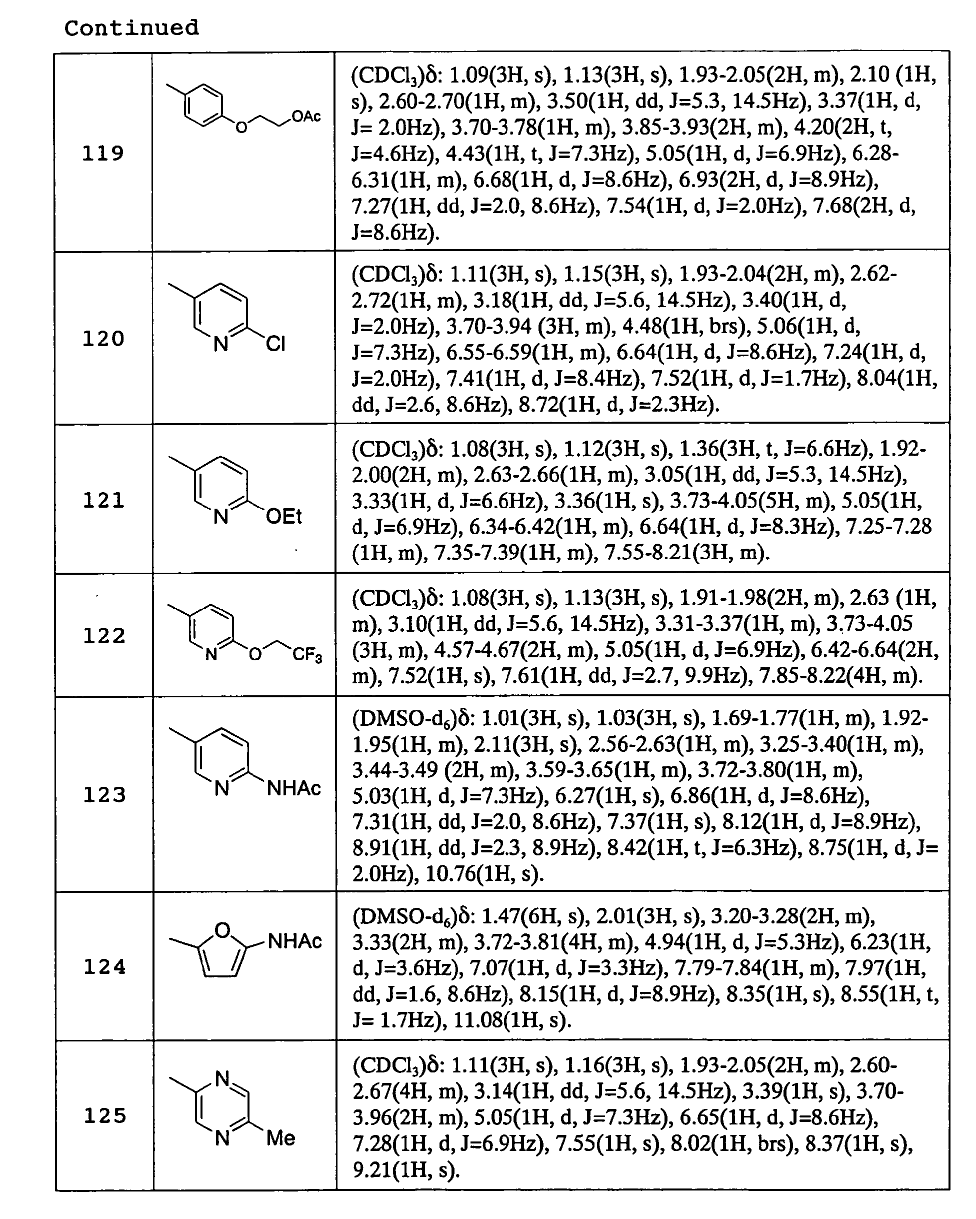

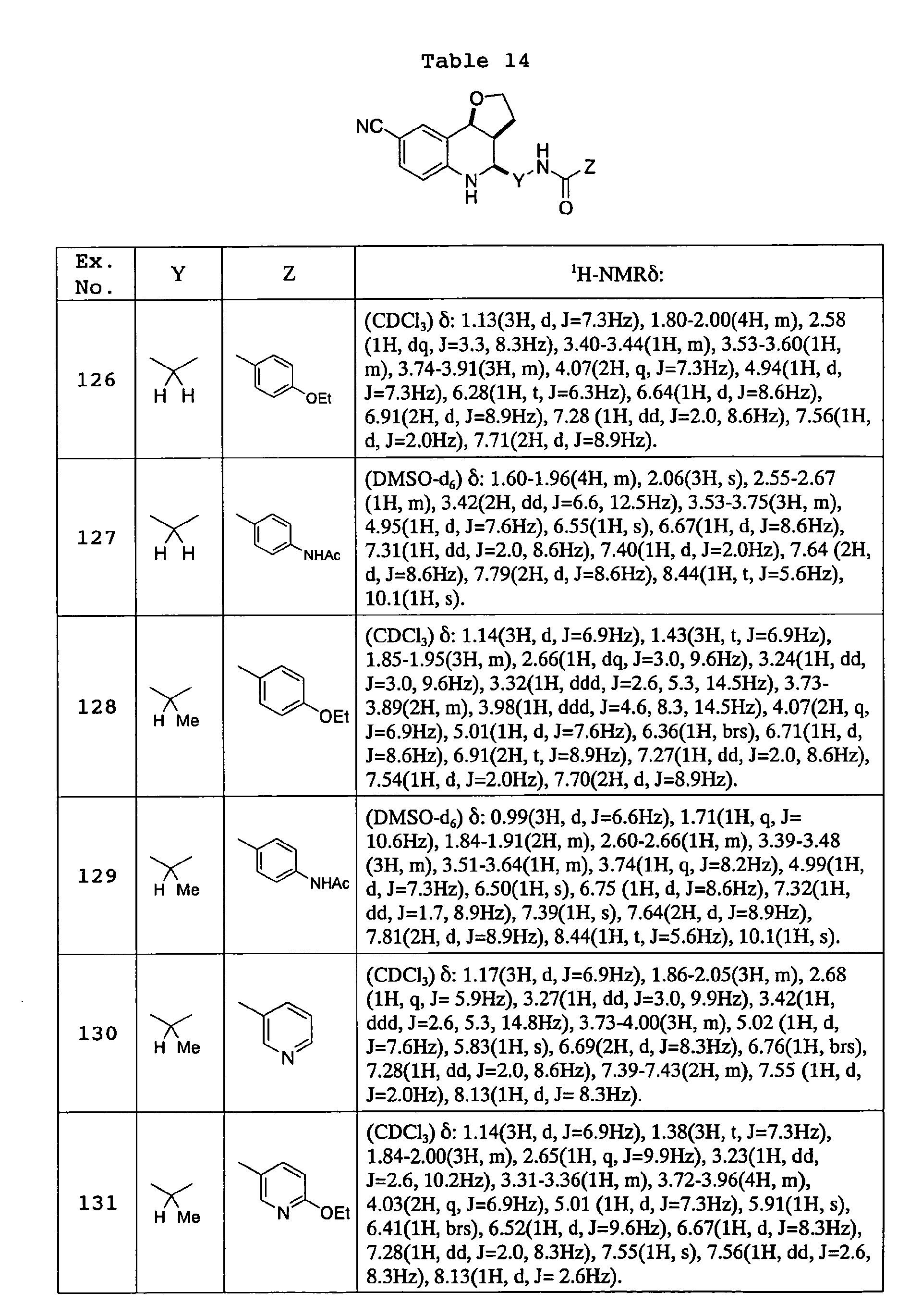

Next, the usefulness of the compounds of the present invention will be described by the following test examples which are by no means intended to limit the scope of the present invention.

### [Test Example 1] Test for competitive binding to rat androgen receptors (rat AR)

Preparation of a rat AR fraction: Prostates were harvested into ice-cooled ET buffer (10 mM Tris, 1 mM EDTA, 5 mM DTT, 10 mM sodium molybdate, pH 7.4) 3 days after orchiectomy in 11-week-old male SD rats. The prostate was finely cut, and ET buffer was added, whereafter the mixture was homogenized using a homogenizer. The homogenate was ultracentrifuged (100,000×g, 60 min, 4°C), and the supernatant was used as a rat AR fraction (hereinafter referred to as ARF).

Binding test: ³H-testosterone (hereinafter referred to as ³H-T) was diluted with ET buffer. Dihydrotestosterone (DHT) was prepared so as to have a final concentration of 1 µM which was 400 times the maximum concentration of ³H-T (2.5 nM). The ³H-T solution was added to a 1.5 ml tube containing DHT, no DHT, or various concentrations of a test compound. Further, 200 µg ARF was added to adjust the final volume to 100 µl. The mixture was incubated for 2 hours at 4°C, and then 300 µl of a 0.05% dextran T70-1.0% activated carbon solution was added. The mixture was further incubated for 15 minutes in ice to remove the free ³H-T by adsorption. After centrifugation (4°C, 2,500 rpm, 5 min), 275 µl of the supernatant was harvested into a liquid scintillation vial, and 2 ml of Clear Sol was added. The mixture was stirred, allowed to stand, and measured for ³H radioactivity with a liquid scintillation counter.

Calculation of the relative binding inhibition rate: The binding inhibition rate (%) of the compound according to the present invention was calculated from the following equation, and the 50% inhibition concentration (IC₅₀) was calculated by the probit analysis of the concentration-binding inhibition curve.

Binding inhibition rate (%) = 100×[1-(a-c)/(b-c)] where
a: Radioactivity of a sample incorporating a compound of the present invention (³H-T+compound)
b: Radioactivity of a sample containing no compound of the present invention (³H-T alone: amount of total binding)
c: Radioactivity of a sample incorporating DHT (³H-T+DHT: amount of nonspecific binding)

The relative binding inhibition rate (RBA: Relative Binding Affinity) was determined from the following equation (Endocrinology 138, 863-870, 1997):
RBA = 100×(IC₅₀ of hydroxyflutamide)/(IC₅₀ of a compound of the present invention)
RBA's of compounds of the present invention, calculated as above, are shown in Table 15.

**Table 15**

| Test Compounds | RBA |
|---|---|
| Example 1 | 1076 |
| Example 12 | 94 |
| Example 20 | 161 |
| Example 26 | 1005 |
| Example 52 | 162 |
| Example 66 | 344 |
| Example 76 | 503 |
| Example 79 | 210 |
| Example 113 | 58 |
| Example 114 | 55 |
| Example 115 | 389 |
| Hydroxyflutamide | 100 |

The RBA's thus determined, with the binding inhibition rate of hydroxyflutamide taken as 100, showed that the compounds of the present invention had very strong binding inhibition activity.

### [Test Example 2] Effect on prostate, levator ani muscle and femoral bone mineral density in orchiectomized (ORX) rats

Testes were removed from 12-week-old male SD rats. Starting on the next day, the compound of Example 1 (3, 10, 30 mg/kg) or DHT (0.1, 1, 10 mg/kg) was subcutaneously administered once daily for four consecutive weeks on a 5-day-a-week basis. The Example compound and DHT were dissolved in dimethyl sulfoxide and diluted 10-fold with olive oil to prepare solutions with various concentrations for use in the test. In an ORX control group, dimethyl sulfoxide as diluted 10-fold with olive oil was used for the test. Rats which had been falsely operated on without orchiectomy were used as a sham control group. On the next day after final administration, the wet weights of the ventral prostate and levator ani muscle were measured and the right femoral bone mineral density was measured by DEXA (double energy X-ray absorption) to evaluate the in vivo efficacy of the compound of Example 1.

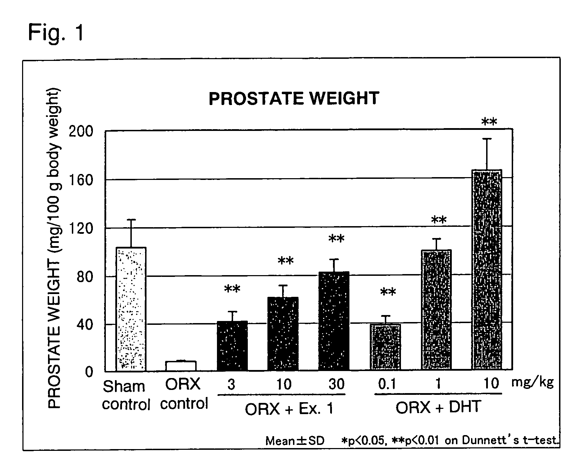

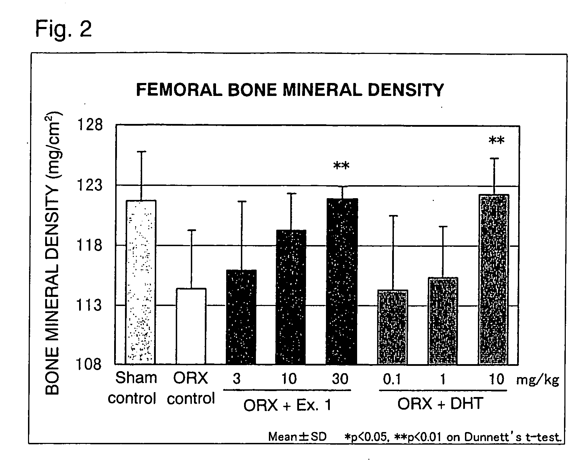

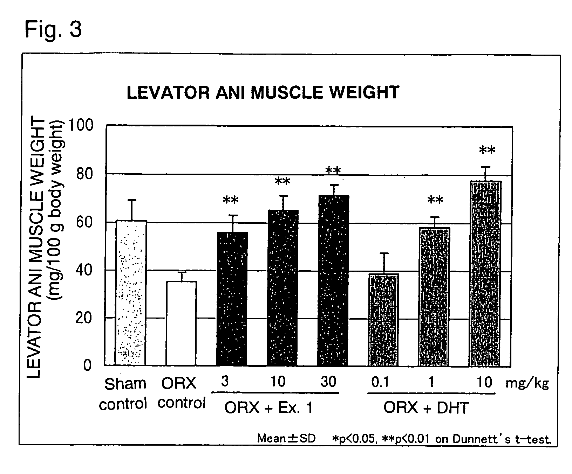

The results are shown in Table 16 and in Figs. 1 to 3.

**Table 16**

| Test compounds | | Weight of prostate (mg/100 g body weight) | Femoral bone mineral density (mg/cm²) | Weight of levator ani muscle (mg/100 g body weight) |
|---|---|---|---|---|
| Sham control | | 103 ± 23 | 122 ± 4 | 61 ± 9 |
| ORX control | | 8 ± 1 | 114 ± 5 | 35 ± 4 |
| ORX+Ex. 1 | 3 mg/kg | 41 ± 8** | 116 ± 6 | 56 ± 7** |
| ORX+Ex. 1 | 10 mg/kg | 61 ± 10** | 119 ± 3 | 65 ± 6** |
| ORX+Ex. 1 | 30 mg/kg | 82 ± 10** | 122 ± 1** | 71 ± 4** |
| ORX+DHT | 0.1 mg/kg | 38 ± 7** | 114 ± 6 | 39 ± 9 |
| ORX+DHT | 1 mg/kg | 100 ± 9** | 115 ± 4 | 58 ± 5** |
| ORX+DHT | 10 mg/kg | 166 ± 26** | 122 ± 3** | 78 ± 6** |
| Mean±SD **p*<0.05, ***p*<0.01 on Dunnett's *t*-test | | | | |

The compound of Example 1 increased the femoral bone mineral density in a dose-dependent manner, exhibiting a significant effect of achieving a comparable level to the sham control group when administered at 30 mg/kg. Similarly, the compound of Example 1 increased the weight of levator ani muscle in a dose-dependent manner, already exhibiting a significant effect at 3 mg/kg. The prostate weight was about 80% of the level of the sham control group at a dose of 30 mg/kg.

When DHT was administered at 10 mg/kg, the femoral bone mineral density and the weight of levator ani muscle increased significantly but, on the other hand, the prostate swelled and its weight increased to about 160% of the level of the sham control group.

From these results, it became clear that the compound of Example 1 does not show an excessive action on the prostate as is observed with natural androgen and that it shows a particularly strong growth action on bone tissue and muscular tissue.

### [Test Example 3] Effect on prostate, levator ani muscle and femoral bone mineral density in orchiectomized (ORX) rats

Testes were removed from 12-week-old male SD rats. Starting on the next day, the compound of Example 12 or 65 (30 mg/kg) or DHT (0.1, 1, 10 mg/kg) was subcutaneously administered once daily for four consecutive weeks on a 5-day-a-week basis. The Example compounds and DHT were dissolved in dimethyl sulfoxide and diluted 10-fold with olive oil to prepare solutions with various concentrations for use in the test. In an ORX control group, dimethyl sulfoxide as diluted 10-fold with olive oil was used for the test. Rats which had been falsely operated on without orchiectomy were used as a sham control group. On the next day after final administration, the wet weights of the ventral prostate and levator ani muscle were measured and the right femoral bone mineral density was measured by DEXA (double energy X-ray absorption) to evaluate the in vivo efficacy of the compounds of Example 12 and 65.

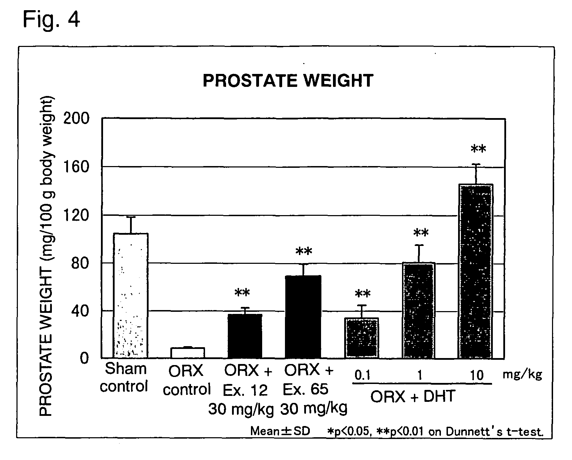

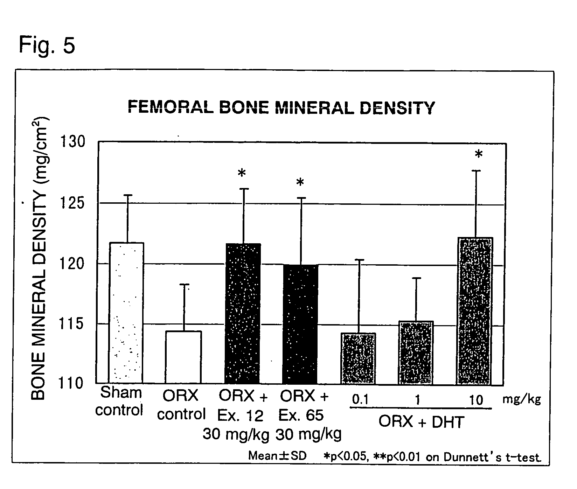

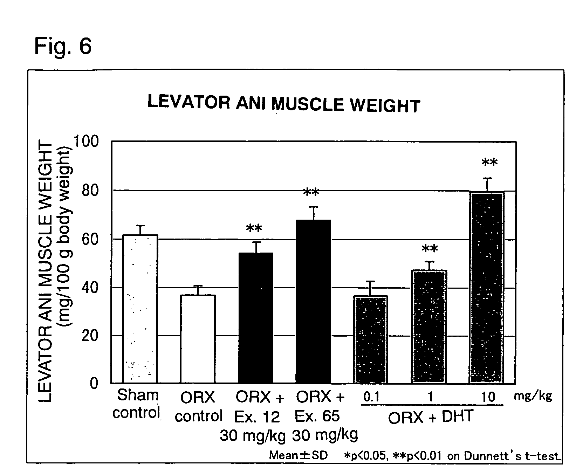

The results are shown in Table 17 and in Figs. 4 to 6.

**Table 17**

| Test compounds | | Weight of prostate (mg/100 g body weight) | Femoral bone mineral density (mg/cm²) | Weight of levator ani muscle (mg/100 g body weight) |
|---|---|---|---|---|
| Sham control | | 104 ± 14 | 121 ± 4 | 61 ± 4 |
| ORX control | | 9 ± 1 | 114 ± 5 | 37 ± 4 |
| ORX+Ex. 12 | 30 mg/kg | 36 ± 6** | 122 ± 5* | 54 ± 5** |
| ORX+Ex. 65 | 30 mg/kg | 69 ± 10** | 120 ± 5* | 67 ± 6** |
| ORX+DHT | 0.1 mg/kg | 34 ± 11** | 114 ± 6 | 37 ± 6 |
| ORX+DHT | 1 mg/kg | 81 ± 15** | 115 ± 4 | 47 ± 3** |
| ORX+DHT | 10 mg/kg | 146 ± 16** | 122 ± 3* | 80 ± 5** |
| Mean±SD **p*<0.05, ***p*<0.01 on Dunnett's *t*-test | | | | |

The compound of Example 12 showed significant effects of increasing the femoral bone mineral density and the weight of levator ani muscle. In this case, the prostate weight was about 35% of the level of the sham control group. The compound of Example 65 also showed significant effects of increasing the femoral bone mineral density and the weight of levator ani muscle. In this case, the prostate weight was about 70% of the level of the sham control group.

In the case of DHT, the femoral bone mineral density increased significantly when it was administered at 10 mg/kg and the weight of levator ani muscle increased significantly at 1 mg/kg; on the other hand, the prostate swelled and its weight increased to about 145% of the level of the sham control group when DHT was administered at 10 mg/kg.

From these results, it became clear that the compounds of Examples 12 and 65 do not show an excessive action on the prostate as is observed with natural androgen and that they show a particularly strong growth action on bone tissue and muscular tissue.

### [Test Example 4] Effect on prostate, levator ani muscle and femoral bone mineral density in orchiectomized (ORX) rats

Testes were removed from 12-week-old male SD rats. Starting on the next day, the compound of Example 113 or 114 (30 mg/kg) or DHT (10 mg/kg) was subcutaneously administered once daily for four consecutive weeks on a 5-day-a-week basis. The Example compounds and DHT were dissolved in dimethyl sulfoxide and diluted 10-fold with olive oil to prepare solutions with various concentrations for use in the test. In an ORX control group, dimethyl sulfoxide as diluted 10-fold with olive oil was used for the test. Rats which had been falsely operated on without orchiectomy were used as a sham control group. On the next day after final administration, the wet weights of the ventral prostate and levator ani muscle were measured and the right femoral bone mineral density was measured by DEXA (double energy X-ray absorption) to evaluate the in vivo efficacy of the compounds of Example 113 and 114.

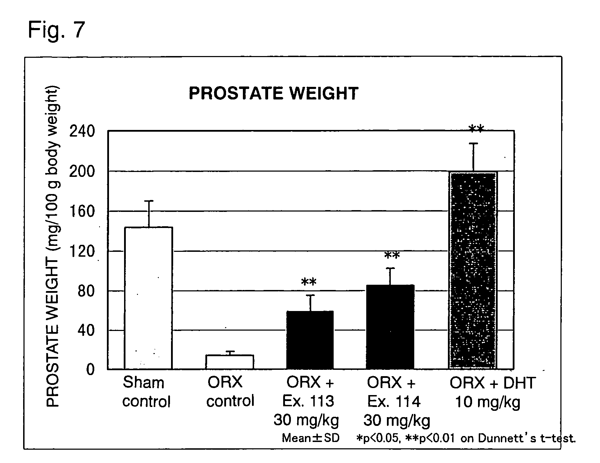

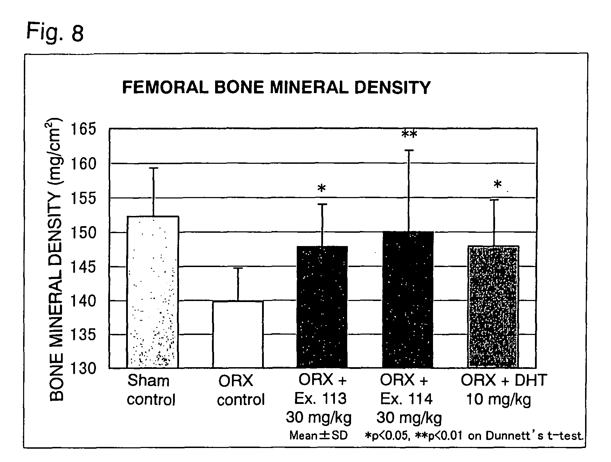

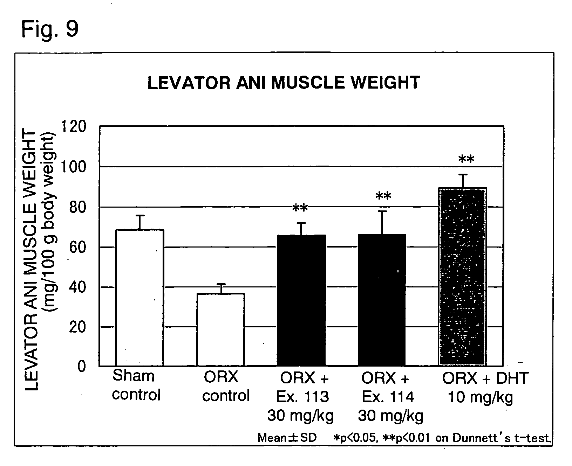

The results are shown in Table 18 and in Figs. 7 to 9.

**Table 18**

| Test compounds | | Weight of prostate (mg/100 g body weight) | Femoral bone mineral density (mg/cm²) | Weight of levator ani muscle (mg/100 g body weight) |
|---|---|---|---|---|
| Sham control | | 144 ± 27 | 152 ± 9 | 68 ± 7 |
| ORX control | | 14 ± 4 | 139 ± 7 | 37 ± 5 |
| ORX+Ex. 113 | 30 mg/kg | 59 ± 16** | 148 ± 3* | 65 ± 6** |
| ORX+Ex. 114 | 30 mg/kg | 86 ± 17** | 150 ± 8** | 66 ± 11** |
| ORX+DHT | 10 mg/kg | 199 ± 27** | 148 ± 5* | 89 ± 7** |
| Mean±SD **p*<0.05, ***p*<0.01 on Dunnett's *t*-test | | | | |

The compound of Example 113 showed significant effects of increasing the femoral bone mineral density and the weight of levator ani muscle. In this case, the prostate weight was about 40% of the level of the sham control group. The compound of Example 114 also showed significant effects of increasing the femoral bone mineral density and the weight of levator ani muscle. In this case, the prostate weight was about 60% of the level of the sham control group.

When DHT was administered at 10 mg/kg, the femoral bone mineral density and the weight of levator ani muscle increased significantly but, on the other hand, the prostate swelled and its weight increased to about 140% of the level of the sham control group.

From these results, it became clear that the compounds of Examples 113 and 114 do not show an excessive action on the prostate as is observed with natural androgen and that they show a particularly strong growth action on bone tissue and muscular tissue.

### [Test Example 5] Effect of oral administration on prostate, levator ani muscle and femoral bone mineral density in orchiectomized (ORX) rats

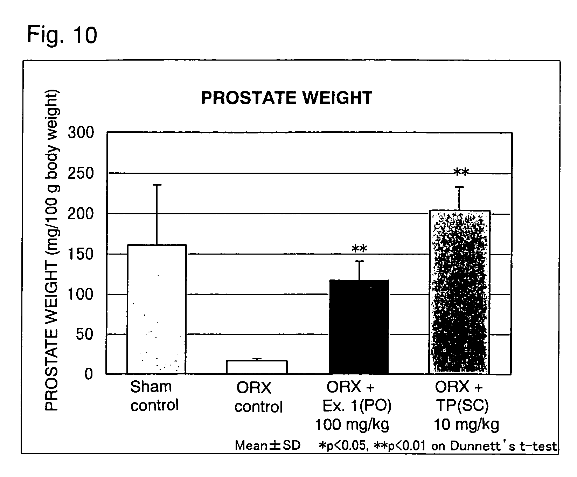

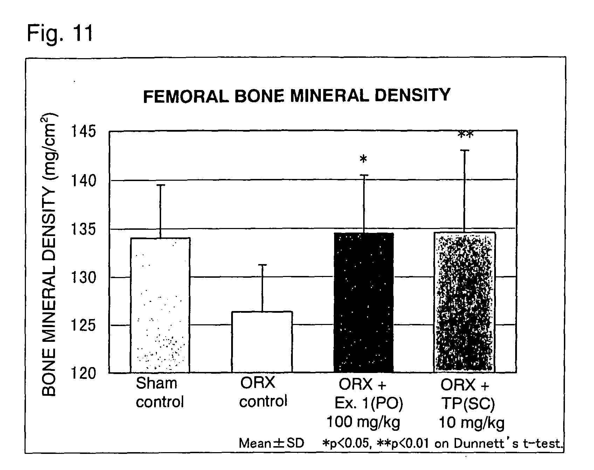

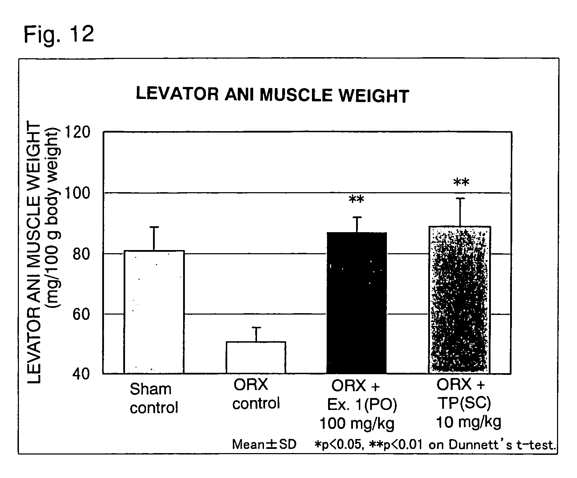

Testes were removed from 12-week-old male SD rats. Starting on the next day, the compound of Example 1 (100 mg/10 ml/kg) was orally administered once daily for four consecutive weeks on a 5-day-a-week basis. As a positive control, testosterone propionate (TP) (10 mg/kg) was subcutaneously administered once daily on a continuous 5-day-a-week basis. The test compound was suspended in a 0.5% methyl cellulose solution used as a solvent whereas TP was dissolved in dimethyl sulfoxide and diluted 10-fold with olive oil for use in the test. In an ORX control group, dimethyl sulfoxide as diluted 10-fold with olive oil was used for the test. Rats which had been falsely operated on without orchiectomy were used as a sham control group. On the next day after final administration, the wet weights of the ventral prostate and levator ani muscle were measured and the right femoral bone mineral density was measured by DEXA (double energy X-ray absorption) to evaluate the in vivo efficacy of the compound of Example 1. The results are shown in Table 19 and in Figs. 10 to 12.

**Table 19**

| Test compounds | | Weight of prostate (mg/100 g body weight) | Femoral bone mineral density (mg/cm²) | Weight of levator ani muscle (mg/100 g body weight) |
|---|---|---|---|---|
| Sham control | | 151 ± 74 | 134 ± 5 | 81 ± 8 |
| ORX control | | 17 ± 3 | 126 ± 5 | 51 ± 5 |
| ORX+Ex. 1(PO) | 100 mg/kg | 117 ± 23** | 135 ± 6* | 87 ± 5** |
| ORX+TP(sc) | 10 mg/kg | 204 ± 29** | 135 ± 8** | 88 ± 9** |
| Mean±SD **p*<0.05, ***p*<0.01 on Dunnett's *t*-test | | | | |

Like TP, the compound of Example 1 significantly increased the femoral bone mineral density and the weight of levator ani muscle in the ORX rats, restoring them to levels comparable to those of the sham control group. The prostate weights of the rats administered the compound of Example 1 were about 75% of the level of the sham control group, whereas TP increased the prostate weight to about 125% of the level of the sham control group.

From these results, it became clear that the compound of Example 1 administered orally gives similar results to those obtained by the aforementioned subcutaneous administration, i.e., it does not show an excessive action on the prostate as is observed with TP but it shows a particularly strong growth action on bone tissue and muscular tissue.

The nonsteroidal AR agonists in current R&D programs are poorly absorbed by the body when administered orally and must be applied as intravenous or intramuscular injection. However, the application of injections imposes burden on patients as by giving them pain or requiring them to see the doctor regularly; therefore, the compound of Example 1 is outstanding in that oral administration is possible without causing burden on the patient.

### [Test Example 6] Effect of oral administration on prostate, levator ani muscle and femoral bone mineral density in orchiectomized (ORX) rats

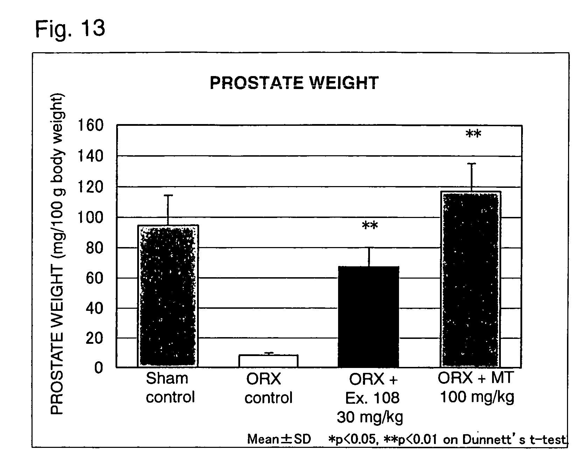

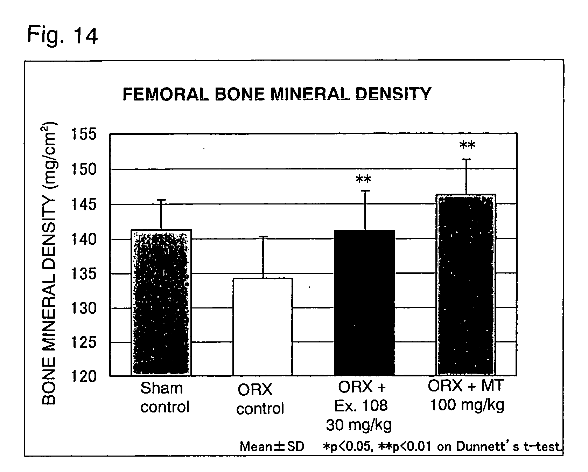

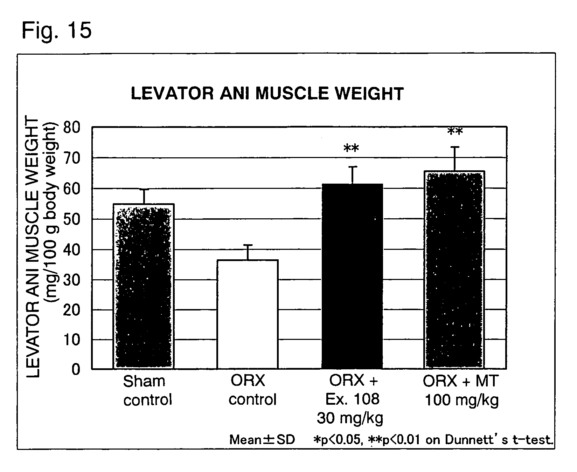

Testes were removed from 12-week-old male SD rats. Starting on the next day, the compound of Example 108 (30 mg/5 ml/kg) was orally administered once daily for four consecutive weeks on a 7-day-a-week basis. As a positive control, methyl testosterone (MT) (100 mg/5 ml/kg) was orally administered once daily on a continuous 7-day-a-week basis. The test compound was suspended in a 0.5% methyl cellulose solution used as a solvent and the suspension was used in the test. In an ORX control group, a 0.5% methyl cellulose solution (5 ml/kg) was administered. Rats which had been falsely operated on without orchiectomy were used as a sham control group. On the next day after final administration, the wet weights of the ventral prostate and levator ani muscle were measured and the right femoral bone mineral density was measured by DEXA (double energy X-ray absorption) to evaluate the in vivo efficacy of the compound of Example 108. The results are shown in Table 20 and in Figs. 13 to 15.

**Table 20**

| Test compounds | | Weight of prostate (mg/100 g body weight) | Femoral bone mineral density (mg/cm²) | Weight of levator ani muscle (mg/100 g body weight) |
|---|---|---|---|---|
| Sham control | | 95 ± 20 | 141 ± 4 | 55 ± 8 |
| ORX control | | 8 ± 2 | 134 ± 6 | 36 ± 5 |
| ORX+Ex. 108 | 30 mg/kg | 67 ± 13** | 141 ± 6** | 61 ± 6** |
| ORX+MT | 100 mg/kg | 117 ± 18** | 146 ± 5** | 65 ± 8** |
| Mean±SD **p*<0.05, ***p*<0.01 on Dunnett's *t*-test | | | | |

Like MT, the compound of Example 108 significantly increased the femoral bone mineral density and the weight of levator ani muscle in the ORX rats, restoring them to levels comparable to those of the sham control group. The prostate weights of the rats administered the compound of Example 108 were about 70% of the level of the sham control group, whereas MT increased the prostate weight to about 120% of the level of the sham control group.

From these results, it became clear that the compound of Example 108 administered orally does not show an excessive action on the prostate as is observed with MT but that it shows a particularly strong growth action on bone tissue and muscular tissue.

The nonsteroidal AR agonists in current R&D programs are poorly absorbed by the body when administered orally and must be applied as intravenous or intramuscular injection. However, the application of injections imposes burden on patients as by giving them pain or requiring them to see the doctor regularly; therefore, the compound of Example 108 is outstanding in that oral administration is possible without causing burden on the patient.

### [Test Example 7] Effect of oral administration on prostate, levator ani muscle and femoral bone mineral density in orchiectomized (ORX) rats

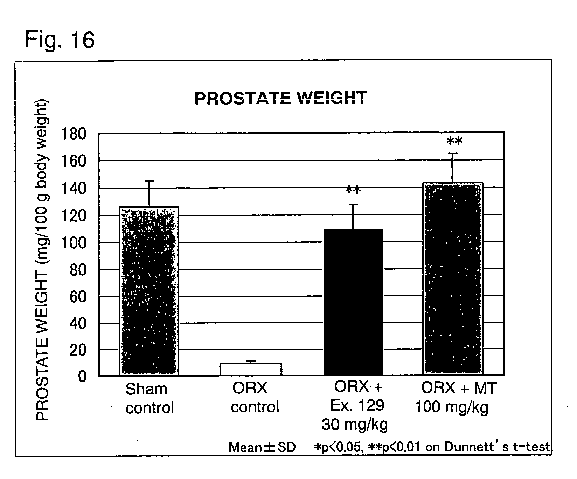

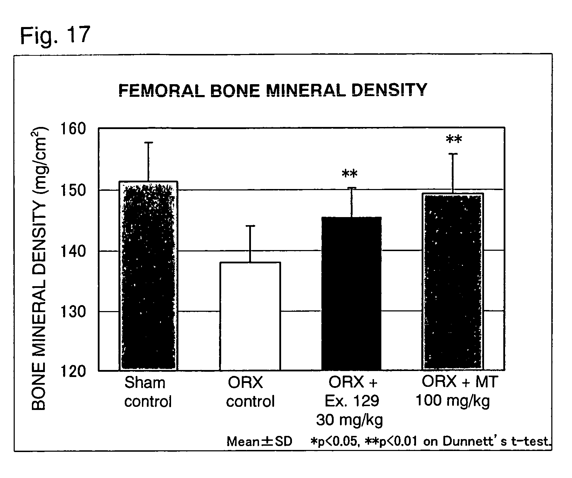

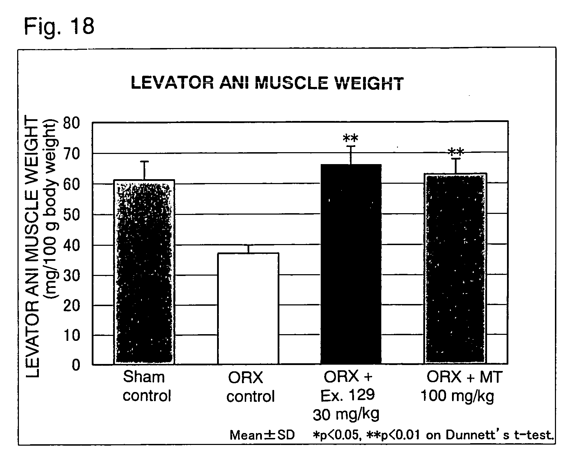

Testes were removed from 12-week-old male SD rats. Starting on the next day, the compound of Example 129 (30 mg/5 ml/kg) was orally administered once daily for four consecutive weeks on a 7-day-a-week basis. As a positive control, methyl testosterone (MT) (100 mg/5 ml/kg) was orally administered once daily on a continuous 7-day-a-week basis. The test compound was suspended in a 0.5% methyl cellulose solution used as a solvent and the suspension was used in the test. In an ORX control group, a 0.5% methyl cellulose solution (5 ml/kg) was administered. Rats which had been falsely operated on without orchiectomy were used as a sham control group. On the next day after final administration, the wet weights of the ventral prostate and levator ani muscle were measured and the right femoral bone mineral density was measured by DEXA (double energy X-ray absorption) to evaluate the in vivo efficacy of the compound of Example 129. The results are shown in Table 21 and in Figs. 16 to 18.

**Table 21**

| Test compounds | | Weight of prostate (mg/100 g body weight) | Femoral bone mineral density (mg/cm²) | Weight of levator ani muscle (mg/100 g body weight) |
|---|---|---|---|---|
| Sham control | | 126 ± 19 | 151 ± 6 | 61 ± 6 |
| ORX control | | 9 ± 2 | 138 ± 6 | 37 ± 3 |
| ORX+Ex. 129 | 30 mg/kg | 109 ± 66** | 145 ± 5** | 66 ± 6** |
| ORX+MT | 100 mg/kg | 143 ± 63** | 149 ± 6** | 63 ± 5** |
| Mean±SD **p*<0.05, ***p*<0.01 on Dunnett's *t*-test | | | | |

Like MT, the compound of Example 129 significantly increased the femoral bone mineral density and the weight of levator ani muscle in the ORX rats, restoring them to levels comparable to those of the sham control group. The prostate weights of the rats administered the compound of Example 129 were about 85% of the level of the sham control group, whereas MT increased the prostate weight to about 115% of the level of the sham control group.

From these results, it became clear that the compound of Example 129 administered orally does not show an excessive action on the prostate as is observed with MT but it shows a particularly strong growth action on bone tissues and muscular tissues.

The nonsteroidal AR agonists in current R&D programs are poorly absorbed by the body when administered orally and must be applied as intravenous or intramuscular injection. However, the application of injections imposes burden on patients as by giving them pain or requiring them to see the doctor regularly; therefore, the compound of Example 129 is outstanding in that oral administration is possible without causing burden on the patient.

Preparation examples of the compound of the present invention will be shown below, but formulations of the compound are not restricted to them.

### [Preparation Example 1] Tablets

In accordance with the following formulation, tablets containing 2 mg of an active ingredient per tablet were prepared:

| | |
|---|---|
| Compound of Example 1 | 2 mg |
| Starch | 48 mg |
| Lactose | 30 mg |
| Cellulose, microcrystalline | 15 mg |
| Methyl cellulose | 3 mg |
| Magnesium stearate | 2 mg |
| Total amount | 100 mg |

### [Preparation Example 2] Capsules

In accordance with the following formulation, 100 mg of an ingredient mixture containing 2 mg of an active ingredient per capsule were encapsulated to prepare capsules:

| | |
|---|---|
| Compound of Example 1 | 2 mg |
| Starch | 38 mg |
| Lactose | 50 mg |
| Cellulose, microcrystalline | 8 mg |
| Magnesium stearate | 2 mg |
| Total | 100 mg |

### INDUSTRIAL APPLICABILITY

The tetrahydroquinoline derivatives of the present invention and pharmaceuticals containing them as active ingredients can exhibit AR agonism while showing none of the excessive actions on the prostate that are observed in androgen steroid preparations. They can also exhibit a particularly strong AR action on skeletal muscle tissue and bone tissue. Hence, the compounds of the present invention can be used in the prevention or treatment of diseases against which various kinds of AR agonism are believed to be effective; in the prevention or treatment of hypogonadism, they can be applied as pharmaceuticals with moderate action on the prostate and with less adverse effects, and in the prevention or treatment of wasting diseases and osteoporosis, they are expected to show potent action on target tissues, such as skeletal muscle tissue and bone tissue.

## Claims

1. A tetrahydroquinoline derivative represented by the following formula (I) or pharmacologically acceptable salts thereof: 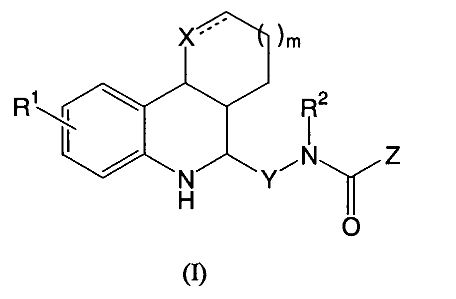 wherein R¹ represents a nitro group or a cyano group;
X represents CH or O, provided that when X is CH, the dashed line represents a double bond;
m represents 0 or 1;
Y represents an alkylene group having 1 - 5 carbon atoms which may be substituted by a substituent selected from the group consisting of an alkyl group having 1 - 5 carbon atoms and a cycloalkyl group having 3 - 7 carbon atoms;
R² represents a hydrogen atom, an alkyl group having 1 - 5 carbon atoms, a cycloalkyl group having 3 - 7 carbon atoms or an aralkyl group having 7 - 9 carbon atoms;
Z represents -B-O-Q
[wherein B represents an alkylene group having 1 - 5 carbon atoms which may be substituted by a substituent selected from the group consisting of an alkyl group having 1 - 5 carbon atoms and a cycloalkyl group having 3 - 7 carbon atoms; Q is a hydrogen atom, an alkyl group having 1 - 5 carbon atoms or a cycloalkyl group having 3 - 7 carbon atoms which may be substituted by a substituent selected from the group consisting of a halogen atom, a hydroxyl group, a cyano group and an alkoxy group having 1 - 5 carbon atoms, or an aryl group, a heteroaryl group or an aralkyl group having 7 - 9 carbon atoms which may have a substituent R³,
R³ represents an alkyl group having 1 - 5 carbon atoms which may be substituted by a fluorine atom, a halogen atom, an aryl group, a heteroaryl group, a nitro group, a cyano group, -A-R⁴ {wherein A represents -CO-, -CO₂-, -COS-, -CONR⁵-, -O-, -OCO-, -OSO₂-, -S-, -SCO-, -SO-, -SO₂-, -NR⁵-, -NR⁵CO-, -NR⁵SO₂-, -NR⁵CONH-, -NR⁵CSNH- or -NR⁵COO- (wherein R⁵ represents a hydrogen atom, an alkyl group having 1 - 5 carbon atoms, a cycloalkyl group having 3 - 7 carbon atoms or an aralkyl group having 7 - 9 carbon atoms), R⁴ is a hydrogen atom, an alkyl group having 1 - 5 carbon atoms which may be substituted by a fluorine atom, a cycloalkyl group having 3 - 7 carbon atoms, a halogen atom, or an aryl group or a heteroaryl group which may be substituted by R⁶ (wherein R⁶ represents an alkyl group having 1 - 5 carbon atoms, an alkoxy group having 1 - 5 carbon atoms or a halogen atom), provided that when A is -NR⁵- or -CONR⁵-, R⁴ and R⁵ may, together with the nitrogen atom to which they are bonded, form pyrrolidine or piperidine)}, or -A'-(CH₂)ₙ-R^{4'} {wherein A' represents a single bond, -CO-, -CO₂-, -COS-, -CONR^{5'}-, -O-, -OCO-, -OSO₂-, -S-, -SCO-, -SO-, -SO₂-, -NR^{5'}-, -NR^{5'}CO-,
-NR^{5'}SO₂-, -NR^{5'}CONH-, -NR^{5'}CSNH- or -NR^{5'}COO- (wherein R^{5'} represents a hydrogen atom, an alkyl group having 1 - 5 carbon atoms, a cycloalkyl group having 3 - 7 carbon atoms or an aralkyl group having 7 - 9 carbon atoms), n represents an integer of 1 or 2, R^{4'} represents a hydrogen atom, an alkyl group having 1 - 5 carbon atoms which may be substituted by a fluorine atom, a cycloalkyl group having 3 - 7 carbon atoms, a halogen atom, a hydroxyl group, a cyano group, an alkoxy group having 1 - 5 carbon atoms, an alkylacyloxy group having 2 - 5 carbon atoms, an alkoxycarbonyl group having 2 - 5 carbon atoms, an aryl group or a heteroaryl group which may be substituted by R^{6'} (wherein R^{6'} represents an alkyl group having 1 - 5 carbon atoms, an alkoxy group having 1 - 5 carbon atoms or a halogen atom), or -NR^{7'}R^{8'} (wherein R^{7'} and R^{8'} each independently have the same meaning as the aforementioned R^{5'}, provided that R^{7'} and R^{8'} may, together with the nitrogen atom to which they are bonded, form pyrrolidine or piperidine), provided that when A' is -NR^{5'}- or -CONR^{5'}-, R^{4'} and R^{5'} may, together with the -N-(CH₂)ₙ- to which they are bonded, form pyrrolidine or piperidine}], or alternatively Z represents -(CH₂)ᵣ-W
[wherein r represents an integer of 0 - 2, W represents a phenyl group having substituent R⁹ at p-position, a naphthyl group which may have substituent R¹⁰ or a heteroaryl group which may be substituted by 1 - 3 independent R¹¹'s (wherein R⁹, R¹⁰ and R¹¹ independently have the same meaning as the aforementioned R³)].

2. The tetrahydroquinoline derivative according to claim 1, where Y is -CH(CH₃)-CH₂- or -C(CH₃)₂-CH₂-, X is CH, m is 0, R² is a hydrogen atom and Z is -CH₂-O-Q (wherein Q represents an alkyl group having 1 - 5 carbon atoms) or pharmacologically acceptable salts thereof.

3. The tetrahydroquinoline derivative according to claim 1, where Y is -CH(CH₃)-CH₂- or -C(CH₃)₂-CH₂-, m is 0, R² is a hydrogen atom and Z is -W [wherein W is a heteroaryl group which may be substituted by 1 - 3 independent R¹¹'s or a phenyl group having substituent R⁹ at p-position {wherein R¹¹ and R⁹ independently represent a halogen atom, an alkyl group having 1 - 5 carbon atoms which may be substituted by a fluorine atom, a nitro group, a cyano group, -A-R⁴ (wherein A is -CO-, -CO₂-, -O-, -NHCO- or -NHCONH-, and R⁴ is a hydrogen atom or an alkyl group having 1 - 5 carbon atoms which may be substituted by a fluorine atom) or -A'-(CH₂)ₙ-R^{4'} (wherein A' is -CO-, -CO₂-, -O-, -NHCO- or -NHCONH-, R^{4'} is a hydrogen atom, an alkyl group having 1 - 5 carbon atoms which may be substituted by a fluorine atom, a hydroxyl group, a halogen atom or an alkoxy group having 1 - 5 carbon atoms, and n is an integer of 1 or 2)}] or pharmacologically acceptable salts thereof.

4. The tetrahydroquinoline derivative according to claim 3, where Z is a phenyl group having substituent R⁹ at p-position or a heteroaryl group having substituent R¹¹ {wherein R⁹ and R¹¹ independently represent a halogen atom, -O-R⁴ or -NHCO-R⁴ (wherein R⁴ represents a hydrogen atom or an alkyl group having 1 - 5 carbon atoms which may be substituted by a fluorine atom)} or pharmacologically acceptable salts thereof.

5. The tetrahydroquinoline derivative according to claim 3, where Z is a phenyl group having substituent R⁹ at p-position or a heteroaryl group having substituent R¹¹ {wherein R⁹ and R¹¹ represent -NHCO-R⁴ (wherein R⁴ represents a hydrogen atom or an alkyl group having 1 - 5 carbon atoms which may be substituted by a fluorine atom)} or pharmacologically acceptable salts thereof.

6. A pharmaceutical comprising the tetrahydroquinoline derivative or pharmacologically acceptable salts thereof according to any one of claims 1 to 5 as an active ingredient.

7. The pharmaceutical according to claim 6, which is an androgen receptor agonist.

8. The pharmaceutical according to claim 7, which can be used in the prevention or treatment of wasting disease or osteoporosis.

9. The pharmaceutical according to claim 7, which can be used in the prevention or treatment of a disease selected from the group consisting of male hypogonadism, male sexual dysfunction, abnormal sex differentiation, male delayed puberty, cancer in female genital organ, breast cancer, mastopathy, endometriosis and female sexual dysfunction.

10. The pharmaceutical according to claim 7, which can be used in the prevention or treatment of hematopoietic dysfunction and diseases related thereto.

11. A method of preventing or treating wasting disease or osteoporosis, which comprises administering to a mammal in need of such prevention or treatment, the tetrahydroquinoline derivative or pharmacologically acceptable salts thereof according to any one of claims 1 to 5 in an amount effective to prevent or treat those diseases.

12. A method of preventing or treating a disease selected from the group consisting of male hypogonadism, male sexual dysfunction, abnormal sex differentiation, male delayed puberty, cancer in female genital organ, breast cancer, mastopathy, endometriosis and female sexual dysfunction, which comprises administering to a mammal in need of such prevention or treatment, the tetrahydroquinoline derivative or pharmacologically acceptable salts thereof according to any one of claims 1 to 5 in an amount effective to prevent or treat those diseases.

13. A method of preventing or treating hematopoietic dysfunction or diseases related thereto, which comprises administering to a mammal in need of such prevention or treatment, the tetrahydroquinoline derivative or pharmacologically acceptable salts thereof according to any one of claims 1 to 5 in an amount effective to prevent or treat those diseases.
